# EUROPEAN PATENT SPECIFICATION

(11) **EP 2 812 349 B1**
(45) Date of publication and mention of the grant of the patent: **01.09.2021**
(21) Application number: 13746774.2
(22) Date of filing: 07.02.2013
(51) Int. Cl.: G01N 33/68

(54) **METHODS FOR THE CHARACTERISATION OF INTERACTION SITES ON TARGET PROTEINS**
VERFAHREN ZUR CHARAKTERISIERUNG VON WECHSELWIRKUNGSBEREICHEN BEI ZIELPROTEINEN
PROCÉDÉ DE CARACTÉRISATION DE SITES D'INTERACTION SUR DES PROTÉINES CIBLES

(30) Priority: 10.02.2012 EP 12154872
(43) Date of publication of application: 17.12.2014
(73) Proprietor: Cambridge Enterprise Limited, Cambridge, Cambridgeshire CB2 1TN (GB)
(72) Inventor: WATT, Paul, Mount Claremont Western Australia 6010 (AU); HARDWICK, Bryn, Cambridge Cambridgeshire CB2 0XZ (GB); McKENZIE, Grahame, Cambridge Cambridgeshire CB2 0XZ (GB); VENKITARAMAN, Ashok, Cambridge Cambridgeshire CB2 0XZ (GB)
(74) Representative: Stratagem IPM Limited
(86) International application number: PCT/AU2013/000110
(87) International publication number: WO 2013/116903

(56) References cited:
- WO-A1-2004/074479
- WO-A1-2005/119244
- WO-A1-2005/119244
- WO-A1-2008/034161
- US-A1- 2004 123 343
- US-B2- 7 517 684
- BARR R K ET AL: "IDENTIFICATION OF THE CRITICAL FEATURES OF A SMALL PEPTIDE INHIBITOR OF JNK ACTIVITY", JOURNAL OF BIOLOGICAL CHEMISTRY, AMERICAN SOCIETY FOR BIOCHEMISTRY AND MOLECULAR BIOLOGY, US, vol. 277, no. 13, 29 March 2002 (2002-03-29), pages 10987-10997, XP001155759, ISSN: 0021-9258, DOI: 10.1074/JBC.M107565200
- PETER MOLEK ET AL: "Peptide Phage Display as a Tool for Drug Discovery: Targeting Membrane Receptors", MOLECULES ONLINE, vol. 16, no. 12, 21 December 2011 (2011-12-21), pages 857-887, XP055224797, DE ISSN: 1433-1373, DOI: 10.3390/molecules16010857
- HAFNER MARKUS ET AL: "Displacement of protein-bound aptamers with small molecules screened by fluorescence polarization", NATURE PROTOCOLS, NATURE PUBLISHING GROUP, GB, vol. 3, no. 4, 1 January 2008 (2008-01-01) , pages 579-587, XP008118421, ISSN: 1750-2799, DOI: 10.1038/NPROT.2008.15 [retrieved on 2008-03-13]
- DATABASE GENBANK 31 December 2011 XP055082789 Database accession no. AES04816
- WATT PM.: 'Phenotypic screening of phylomer peptide libraries derived from genome fragments to identify and validate new targets and therapeutics.' FUTURE MED CHEM. vol. 1, no. 2, May 2009, pages 257 - 265, XP055078983
- WATT PM ET AL.: 'Protein silencing with Phylomers: a new tool for target validation and generating lead biologicals targeting protein interactions.' EXPERT OPIN DRUG DISCOV. vol. 1, no. 5, October 2006, pages 491 - 502, XP008174501
- WATT PM.: 'Screening for peptide drugs from the natural repertoire of biodiverse protein folds.' NAT BIOTECHNOL. vol. 24, no. 2, February 2006, pages 177 - 183, XP002555147
- BAINES I C ET AL: "Peptide aptamers as guides for small-molecule drug discovery", DRUG DISCOVERY TODAY, ELSEVIER, AMSTERDAM, NL, vol. 11, no. 7-8 1 April 2006 (2006-04-01), pages 334-341, XP027889175, ISSN: 1359-6446 [retrieved on 2006-04-01]
- PIERRE COLAS: "The eleven-year switch of peptide aptamers", JOURNAL OF BIOLOGY, vol. 7, no. 1, 31 January 2008 (2008-01-31), page 2, XP055498976, GB ISSN: 1475-4924, DOI: 10.1186/jbiol64

## Description

The present invention relates to improved and integrated methods for the characterisation of an interaction site on a target protein that modulates the phenotype of a mammalian cell, such as a phenotype other than death and/or reduced growth. Such methods of the present invention identify a target protein that modulates such a phenotype of a mammalian cell, and characterise an interaction site on said target protein as dockable by a small molecule ligand. Such identification and characterisation methods are useful in the development of research tools and/or therapeutics, such as protein/peptide or small molecule therapeutics. Accordingly, the present invention also relates to identification of a small molecule ligand, that binds to such a target protein. Also described herein is identification a compound being a candidate modulator of said phenotype of a mammalian cell. Described herein are peptides or proteins, or fragments, variants and/or derivatives thereof, comprising certain amino acid sequences, nucleic acids encoding such peptides or proteins and uses of such peptides or proteins or of such nucleic acids.

Virtually all biological process or phenotypes of mammalian cells, particularly those involved in disease-related phenotypes, are regulated by cellular pathways; which in turn are typically regulated through the interactions between biological macromolecules such as proteins. The multiplicity of such interactions involved in any given biological process or phenotype, together with the lack of generally applicable tools for their investigation and/or manipulation, presents a formidable challenge to current strategies for functional genomics, chemical biology and discovery of candidate therapeutics. Identifying molecules such as biologically active peptides or small molecules that bind to, and/or modulate interactions between, proteins involved in biological process or phenotypes of mammalian cells represents a valuable approach to such problems.

Over-expression of peptide libraries have been used in trans-dominant effector screens to identify protein:protein interactions that regulate specific cellular phenotypes (Xu et al., 2001, Nat Genet 27: 23-29; WO 1997/27212). Moreover, synthetic peptides have been successfully used as biologic therapeutics to modulate the functions or interactions of proteins (Leader et al., 2008, Nat Rev Drug Discov 7: 21-29). Thus, biologically active peptides represent important tools for the identification and therapeutic manipulation of novel cellular targets. However, currently available methods for their identification are notoriously inefficient, placing target-directed or phenotypic screens at the limits of feasibility. For example, hit-rates as low as 1 in 1,000,000 have been reported in library screens using random peptides (Colas et al., 1996, Nature 380: 548-550; Park and Raines, 2000 Nat Biotechnol 18: 847-851; Xu and Luo, 2002, Oncogene 21: 5753-5757; Xu et al., 2001). These low hit rates have been variously attributed to the random nature of the peptide sequence employed, or to the use of constrained scaffolds that restrain secondary structure formation. These factors may severely limit the complexity of the chemical 'space' that can effectively be surveyed by peptide libraries, underscoring the need for alternative approaches.

Certain functional genomics or chemical biology technologies have been reported that enable the identity of a target protein to be determined; given that a molecule is known that binds to the target. For example, as described in Daub et al., 2004 (Assay Drug Dev Technol 2: 215-224), Brehmer et al., 2005 (Cancer Res 65: 379-382) and WO 2004/013633. However, such methods require that such known binding molecule is one having sufficiently high affinity and specificity to the target protein such that it can be isolated and hence identified by such procedures.

Once a target protein and a binding molecule have been identified, other technologies may be available to investigate the site of interaction between these two entities. For example, as described in Chan et al. (2011, Cancer Cell, 19 435-437), Begley et al., (2011, J Struct Funct Genomics, 12: 63-76), Staker et al., (2005, J Med Chem 48: 2336-2345), Di Paolo et al., (2010, Nature Chem Biol [Epub ahead of print] PMID: 21113169) and Petros et al., (2000, Prot Sci, 9: 2528-2534). However, in the absence of information on the phenotypic relevance of the binding, and with only a limited number of binding molecule typically available for each target protein or interaction site

(especially of those binding molecules known to modulate a phenotype of interest of relevance to the target protein), the degree - and hence usefulness - of the data derived from such limited-scale investigations of the interaction site on the target protein, often does not provide characterisation of the interaction site that is sufficient to enable efficient discovery or research of drug candidates that modulate the desired phenotype.

Protein folds in higher organisms are proposed to have evolved from the assembly of 'antecedent domain segments', 15-30 amino acid elements found throughout Eubacterial and Archaeal genomes that are thought to specify selective functions (for example protein:protein interaction), sometimes in the absence of an intrinsic tertiary structure (Bogarad and Deem, 1999, PNAS 96: 2591-2595; Gilbert et al., 1997, PNAS 94: 7686-7703; Lupas et al., 2001, J Struct Biol 134: 191-203; Riechmann and Winter, 2000, PNAS 97: 10068-10073; Riechmann and Winter, 2006, J Mol Biol 363: 460-468; Soding and Lupas, 2003, Bioessays 25: 837-846). Phylogenetically-diverse gene fragments derived from natural Eubacterial and Archaeal have been hypothesised to provide a rich source of bioactive peptides, offering an improved alternative to existing random and/or constrained peptide libraries (Watt, 2006, Nat Biotech 24: 177-183), and libraries of nucleic acids encoding such peptides have been produced for screening (Watt et al., 2006, Expert Opin Drug Disc 1: 491-502; Watt et al., 2009, Future Med Chem 1: 257-265; WO 2000/68373; WO 2004/074479; WO 2006/017913; WO 2007/097923).

The examples of WO 2005/119244 describe the uses of libraries of phylogenetically-diverse gene fragments derived from natural Eubacterial and Archaeal to screen for and identify peptides that rescue cell death or prevent reduced growth of certain mammalian cells. Screening for such rescue from death and/or increased growth of mammalian cells would, in the light of the current state of the art, be presumed possible to the person of ordinary skill using libraries of peptides even with those screens/libraries with low hit-rates, as such selections are implicitly able to overcome such issues; since only the positive hits create a data point (living cell/cell colony) that requires further analysis in the screen.

Watt et al., 2009 (Future Med Chem 1: 257-265) describes phenotypic screening of Phylomer libraries derived from genome fragments to identify new targets and therapeutics.

Watt et al., 2006 (Expert Opin Drug Disc 1: 491-502) describes protein silencing with Phylomer as a tool for target validation and generating lead biologicals targeting protein interactions.

Barr et al., 2002 (J Biol Chem 277: 10987-10997) describes the identification of critical features of a small peptide inhibitor of JNK activity.

Molek et al., 2011 (Molecules Online 16: 857-887) describes peptide phage display as a tool for drug discovery by targeting membrane receptors.

Hafner et al., 2008 (Nat Prot 3: 1850-2799) describes displacement of protein-bound aptamers with small molecules screened by fluorescence polarisation.

Baines et al., 2006 (Drug Disc Today 11: 334-341) describes peptide aptamers as guides for small molecule drug design.

Colas, 2008 (J Biol 7: 2) describes certain uses of peptide aptamers, including also as guides for small molecule drug design.

Summarising the above, the prior art does not provide efficient and/or integrated methods to enable the characterisation of an interaction site of a target protein as dockable by a small molecule ligand, wherein the target modulates a (desired) phenotype of a mammalian cell as claimed herein. Furthermore, the above methods do not provide or utilise a particular biological library or peptide-class resource suitable for use in a number of key steps of such methods. The use of such a particular biological library or peptide-class resource provides particular advantages in terms or one or more of: reduced resources and technological expertise required at such steps; screening cost and efficiency through increased hit-rate and/or affinity; flexibility and efficiency in investigating diverse cell-types, phenotypes, target-types and/or interaction sites. In particular, the above prior art does not provide efficient methods or tools - for example by the provision of utilisation of a particular biological library or peptide-class resource - to transition the results of the (desired) phenotype screen into methods required for the identification of drug candidates, particularly small-molecule drug candidates.

Accordingly, it is an object of the present invention to provide alternative, improved and/or integrated means or methods that address one or more of these problems.

The present invention is set out in the appended set of claims.

The present invention relates to **a method of characterising an interaction site on a target protein,** as dockable by a small molecule ligand, wherein the target protein modulates a phenotype of a mammalian cell, said method comprising the steps:
exposing a population of *in-vitro* cultured mammalian cells capable of displaying said phenotype to a library of peptides of about 8 to about 180 amino acids encoded by nucleic acid fragments obtainable from genome(s) of a microorganisms and/or a eukaryotic species having a compact genome;
identifying a cell in the population which displays an alteration in said phenotype following said exposure;
identifying a peptide from said library of peptides that alters said phenotype of the cell;
providing the identified peptide;
identifying a cellular protein which binds to said provided identified peptide, said cellular protein being a target protein which modulates said phenotype of the mammalian cell;
providing said target protein;
providing a population of peptides of about 8 to about 180 amino acids encoded by nucleic acid fragments obtainable from genome(s) of a microorganisms and/or a eukaryotic species having a compact genome, wherein the peptides in said population bind to said target protein at the interaction site, wherein, prior to its provision, said population of peptides is identified by screening a library of peptides of about 8 to about 180 amino acids encoded by nucleic acid fragments obtainable from genome(s) of a microorganisms and/or a eukaryotic species having a compact genome, or of nucleic acids that encode peptides of about 8 to about 180 amino acids encoded by nucleic acid fragments obtainable from genome(s) of a microorganisms and/or a eukaryotic species having a compact genome, for binding of said encoded peptides to said target protein;
empirically determining the binding configuration of a plurality of binding peptides, within said population that has different sequences, to said target protein, wherein at least one of said binding peptides modulates the phenotype in a mammalian cell capable of displaying the phenotype; and
identifying: (i) locations of binding energy; and/or (ii) the orientation and identity of at least one side chain of said plurality of binding peptides that interacts with said protein target, in either case characterising the three dimensional structure of said interaction site by analysis of said binding configuration, wherein said three dimensional structure is characterised using *in silico* methods, and
   (x) docking the structure of a small molecule to the characterised interaction site *in silico;* or
   (y) employing at least one of said binding peptides and the target protein in an assay to identify a small molecule that modulates the binding of the peptide to the target protein at the interaction site,
thereby characterising the interaction site on said target protein as dockable by a small molecule ligand.

Described herein is **a method of identifying a target protein** which modulates the phenotype of a mammalian cell, such as a phenotype other than death and/or reduced growth , said method comprising the steps:
- exposing a population of in-vitro cultured mammalian cells capable of displaying said phenotype to a library of Phylomers;
- identifying a cell in the population which displays an alteration in said phenotype following said exposure;
- identifying a Phylomer that alters said phenotype of the cell;
- providing the identified Phylomer; and
- identifying a cellular protein which binds to said provided Phylomer, said cellular protein being a target protein which modulates said phenotype of the mammalian cell.

Described herein is **a method of characterising an interaction site on a target protein** which modulates the phenotype of a mammalian cell, such as a phenotype other than death and/or reduced growth, said method comprising the steps:
- providing said target protein;
- providing a population of Phylomers which bind to said target protein;
- empirically determining the binding configuration of at least one Phylomer within said population to said target protein; and
- identifying: (i) locations of binding energy; and/or (ii) and/or (ii) the orientation of at least one side chain of said Phylomer that interacts with said protein target, in either case by analysis of said binding configuration,
thereby characterising the interaction site on said target protein.

Described herein is **a method of identifying a ligand** which binds to a target protein, wherein the target protein modulates the phenotype of a mammalian cell, such as a phenotype other than death and/or reduced growth, said method comprising the step of identifying, using in silico methods, the structure of a ligand which is dockable to a three dimensional structure of an interaction site of said target protein, wherein said three dimensional structure is determined by a method described herein.

Described herein is **a method of identifying a compound** which is a candidate modulator of the phenotype of a mammalian cell, such as a phenotype other than death and/or reduced growth, said method comprising the steps:
- exposing a population of *in-vitro* cultured mammalian cells capable of displaying said phenotype to a library of Phylomers;
- identifying a cell in the population which displays an alteration in said phenotype following said exposure;
- identifying a Phylomer that alters said phenotype of the cell;
- providing the identified Phylomer;
- identifying a cellular protein which binds to said provided Phylomer, said cellular protein being a target protein which modulates said phenotype of the mammalian cell;
- providing said target protein and said provided Phylomer; and
- determining the effect of a test compound on the binding of said Phylomer to said target protein,
wherein a test compound which modulates the degree of binding of said Phylomer to said target protein is a candidate modulator of said phenotype of the mammalian cell.

Described herein is **a method of identifying Phylomer** which modulates the phenotype of a mammalian cell, such as a phenotype other than death and/or reduced growth, said method comprising the steps: exposing a population of *in-vitro* cultured mammalian cells capable of displaying said phenotype to a library of Phylomers; identifying a cell in the population which displays an alteration in said phenotype following said exposure; and identifying a Phylomer that alters said phenotype of the cell, said Phylomer being one which modulates said phenotype of the mammalian cell.

Described herein is **a peptide or protein** comprising the amino acid sequence of a peptide identified by a method described herein, including one selected from the list consisting or: 4G9, 6F6, 6G8, 10B11, 25C3, 44B2 and 48E6, or a fragment, variant and/or derivative of said peptide or protein, and to the **use of the peptide or protein,** or a fragment, variant and/or derivative thereof, to: (i) modulate a phenotype of a mammalian cell, other than death and/or reduced growth; and/or (ii) to bind to a target protein that modulates a phenotype of a mammalian cell, other than death and/or reduced growth. Also described herein is **a nucleic acid** encoding such peptide or protein, or a fragment, variant and/or derivative thereof.

The figures show:
FIGURE 1 depicts the results of the primary phenotypic screen showing the luminescence generated with each Phylomer as a percentage of the plate mean. Negative control vector (pcDNA3.1/nV5-DEST-GusB) and positive control PYC36 are shown along with their overall statistics for the entire screen across 50 plates.
FIGURE 2 depicts secondary validation of 14 Phylomers from the primary screen. 7 clones showed significant (n=3, p <0.05 denoted by *) inhibition of PMA-induced API activity; luciferase expression is normalised to Renilla luminescence.
FIGURE 3 depicts Phylomer validation against Srxn1-luciferase activity showed PYC36 and 3 additional Phylomers demonstrated significant inhibition of PMA-induced promoter activity (n=3, p <0.05 denoted by *) (FIGURE 3a). These 3 Phylomers show no significant effect on Srxn1 promoter containing mutated API response elements (n=3, p >0.05) (FIGURE 3b); in each case, data shown as fold induction of normalised luminescence compared to control (Renilla) vector.
FIGURE 4 depicts results of the immunoprecipitation experiment showing the interaction between V5-25C3 Phylomer and Flag-CNH. HEK293T cells were transfected with V5-tagged 25C3 alone or with flag-tagged CNH domain of MAP4K4. Cell lysates were prepared in NP-40 buffer and immunoprecipitated with Mouse V5 antibody and immunoblotted with mouse Flag M2 antibody. As a control, cells were also transfected with flag-CNH alone and immunoprecipitated with Flag-M2 and immunoblotted with the same antibody (Flag M2).
FIGURE 5 depicts the results of the binding experiment of immobilised MBP- MAP4K4-CNH (refolded from the insoluble fraction; 45µg/ml) binds strongly with known ligand RAP2A and with Phylomer 25C3, and marginally or not at all with specificity controls (irrelevant Phylomer PYC35 and the MBP fusion protein). All ligands expressed as MBP fusions and tested at 1uM.
FIGURE 6 depicts the results of Octet-Red biolayer interferometry showing titration of 25C3 (FIGURE 6a) and RAP2A (FIGURE 6b) (100-1000 mM) against immobilised MBP-MAP4K4-CNH (soluble fraction, 45µg/ml).
FIGURE 7 depicts the results of cells transfected with either 25C3 or empty pcDNA3.1 vector (control) scratch-wounded 24 hours later, and wound closure analysed using time-lapse microscopy. Representative time-lapse images at 0, 10 h and 15 h post-wounding are shown (FIGURE 7a; bar = 100 um), and wound widths measured across 4 scratches for 25C3 and control over this time course (FIGURE 7b; * p=<0.001).
FIGURE 8 depicts the amino acid sequences (and corresponding encoding nucleotide sequences) for certain API-inhibitory Phylomers identified by a phenotype screen described herein.
FIGURE 9 depicts a flow-chart with one possible use of methods described herein for target discovery and determination of a binding interface.
FIGURE 10 depicts in graphical form, possible steps and particular technologies that may be employed when using of methods described herein for exploring (cell) phenotype (binding) phamacophore.
FIGURE 11 depicts in graphical form, high-throughput fluorescence polarisation using of Phylomers as probes for screening for small molecule ligands that bind target protein, in particular at an interaction site characterised using methods described herein.

The present invention, and particular non-limiting aspects and/or embodiments thereof, can be generally described as follows:
The present invention relates to methods of characterising an interaction site on a target protein as dockable by a small molecule ligand, wherein the target protein is involved in the modulation of a phenotype of a mammalian cell. Accordingly, the present invention relates to **a method of characterising an interaction site on a target protein** as dockable by a small molecule ligand, wherein the target protein modulates a phenotype of a mammalian cell, said method comprising the steps:
exposing a population of *in-vitro* cultured mammalian cells capable of displaying said phenotype to a library of peptides of about 8 to about 180 amino acids encoded by nucleic acid fragments obtainable from genome(s) of a microorganisms and/or a eukaryotic species having a compact genome;
identifying a cell in the population which displays an alteration in said phenotype following said exposure;
identifying a peptide from said library of peptides that alters said phenotype of the cell;
providing the identified peptide;
identifying a cellular protein which binds to said provided identified peptide, said cellular protein being a target protein which modulates said phenotype of the mammalian cell;
providing said target protein;
providing a population of peptides of about 8 to about 180 amino acids encoded by nucleic acid fragments obtainable from genome(s) of a microorganisms and/or a eukaryotic species having a compact genome, wherein the peptides in said population bind to said target protein at the interaction site, wherein, prior to its provision, said population of peptides is identified by screening a library of peptides of about 8 to about 180 amino acids encoded by nucleic acid fragments obtainable from genome(s) of a microorganisms and/or a eukaryotic species having a compact genome, or of nucleic acids that encode peptides of about 8 to about 180 amino acids encoded by nucleic acid fragments obtainable from genome(s) of a microorganisms and/or a eukaryotic species having a compact genome, for binding of said encoded peptides to said target protein;
empirically determining the binding configuration of a plurality of binding peptides, within said population that has different sequences, to said target protein, wherein at least one of said binding peptides modulates the phenotype in a mammalian cell capable of displaying the phenotype; and
identifying: (i) locations of binding energy; and/or (ii) the orientation and identity of at least one side chain of said plurality of binding peptides that interacts with said protein target, in either case characterising the three dimensional structure of said interaction site by analysis of said binding configuration, wherein said three dimensional structure is characterised using *in silico* methods, and
   (x) docking the structure of a small molecule to the characterised interaction site *in silico;* or
   (y) employing at least one of said binding peptides and the target protein in an assay to identify a small molecule that modulates the binding of the peptide to the target protein at the interaction site,
   thereby characterising the interaction site on said target protein as dockable by a small molecule ligand.

Terms as set forth herein are generally to be understood by their common meaning unless indicated otherwise. Where the term "comprising" or "comprising of" is used herein, it does not exclude other elements. For the purposes of this description, the term "consisting of" is considered to be a particular embodiment of the term "comprising of'. If hereinafter a group is defined to comprise at least a certain number of embodiments, this is also to be understood to disclose a group that consists of all and/or only of these embodiments. Where used herein, "and/or" is to be taken as specific disclosure of each of the two specified features or components with or without the other. For example "A and/or B" is to be taken as specific disclosure of each of (i) A, (ii) B and (iii) A and B, just as if each is set out individually herein. In the context of this description, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect.

Activity of the target protein, such as abnormal function or disfunction (or controlled activity brought about by a stimulus), alters one or more characteristic of the cell, such as a phenotypic characteristic. For example, activation or expression and/or inhibition or suppression of the target protein alters the phenotype of the mammalian cell. Such alteration may be in a positive or negative direction. Moreover, an alteration in the phenotype in one direction or other may be achieved through addition of hormones, growth factors mitogens, chemokines or cytokines or through the infection of cells by a microbe such as a bacterium fungus or virus. Such phenotypes could be reversed either partially, through the action of a compound such as a small molecule, and in particular through the action of a Phylomer peptide for example one from a Phylomer library. For example, the degree of the characteristic associated with the phenotype may become more or less detectable upon its alteration. Any target protein, the presence, absence or activity of which is associated with the alteration of the degree of such a characteristic can be considered to be a target protein involved in the modulation of a phenotype of the respective cell.

Suitable target proteins may, for example, be components of a cellular signalling pathway. Cell signalling pathways are series of interacting factors in a cell that transmit signals within (or from/to the surface off) the cell in response to one or more stimuli, for example external stimuli arising at or in contact with the cell surface, leading to some detectable alteration in the cell's phenotype. Signals transmitted by cell signalling pathways may for example result in activation of transcription factors that alter gene expression in the cell. Preferred cell signalling pathways are active in diseased cells. For example a pathway may be constitutively activated (i.e. permanently switched on) in a cancer cell, or inappropriately activated by an extracellular ligand, for example, in an inflammatory cell in the context of rheumatoid arthritis.

A target protein involved in the modulation of a cellular phenotype may be a putative drug target. Pharmaceutical modulation of the activity of the target protein, for example by binding to an interaction site and blocking interaction to its cellular binding partners (such as those that form part of the a cellular signalling pathway), may alter a cell phenotype in a manner that seeks to achieve a therapeutic effect.

Any mammalian cells that are culturable, that is those which can be maintained or propagated *in-vitro,* may be employed. Such cells may be stable cell lines, such as those obtainable from ATCC or other cell-repositories. Alternatively, the mammalian cells employed may be primary cells derived from a tissue or organ of an individual organism. In certain embodiments, the mammalian cells employed may be transiently transfected with genetic constructs, such as those involved in the (desired) phenotype, for example a reporter gene. In other embodiments, the mammalian cells may be infected with a virus or bacterium which leads to an alteration in the phenotype. If the mammalian cells can be maintained or propagated for the period of the desired assay, they may be employed as described herein. Of particular utility are mammalian cells are those selected from the list consisting of: human cells, murine cells, hamster cells, rate cells, primate cells, and cells from a domestic mammals (such as ovine, bovine, equine, canine or feline cells). Particular cells for employment as described herein include mammalian cells derived from a cancer or tumour, or those associated with a disease or abnormality of a mammal.

Suitable mammalian cells are those that are capable of displaying, or that do display, a phenotype (such as one described herein) the alteration of which is desired to be monitored for modulation. For example, cells may display a phenotype whose inhibition within the assay is to be determined, or cells may not display a phenotype whose stimulation within the assay is to be determined. Mammalian cells which display the appropriate phenotypic characteristics (phenotype) may be identified or obtained by any convenient technique or source, including those known to the person of ordinary skill in the art. For example, cells with wild-type p53 sequence may be useful in screening for phenotypes associated with or being the reactivation of p53-dependent apoptosis.

In particular embodiments, the phenotype to be monitored for modulation may be one having been specifically engineered for a given cell type. For example, and as described in one particular embodiment in the examples herein, a mammalian cell type may be recombinantly engineered to express a phenotype that is convenient or suitable for detection, such as a fluorescent protein/marker (such as GFP; eGFP, and other fluorescent proteins as will be known to the person of ordinary skill), a luminescent protein/marker (such as luciferase) or a cell-surface marker than is detectable with a labelled antibody.

In certain embodiments, the phenotype of the mammalian cell is: one associated with a cell signalling pathway, preferably an activated cell signalling pathway; and/or_one selected from the list consisting of: luminescence, fluorescence, viability, senescence, differentiation, migration, invasion, chemotaxis, apoptosis, immunological anergy, surface marker expression, progress through the cell cycle, transcriptional activity, protein expression, glycosylation, resistance to infection, permeability and reporter-gene activity. In particular of such embodiments, the phenotype is not death and/or reduced (or decreased) growth, such as a phenotype that is not rescue of a cell from cytokine dependence, is not rescue from apoptosis (including neutrophil apoptosis/cell-death), is not induction of colony formation, or is not a rescue screen. For example, a screen for such a phenotype may be designed to detect a trait or characteristic of the cell that is not a rescue from cell death or an increase in growth of the mammalian cell.

In some embodiments, the mammalian cells may display a phenotype that is associated with an activated cell signalling pathway, or alternatively one associated with a supressed (eg inactivated) cell signalling pathway.

A cell signalling pathway of interest may be constitutively activated in the mammalian cells i.e. the signalling pathway is permanently switched on and active in the cell. For example, a mammalian cell may have a mutation, preferably in an upstream pathway component of the pathway, such as a cell surface receptor, which causes constitutive activation of the pathway. Suitable mammalian cell lines with constitutively active cell signalling pathways are well known in the art.

Alternatively, a cell may be transfected with a mutant component of the pathway which causes constitutive activation of the pathway or the cell signalling pathway of interest may be activated in the cell using a drug or the natural ligand. For example, recombinant snonic hedgehog protein may be used to activate hedgehog signalling.

Various aspects of the present description employ Phylomers, libraries of Phylomers or nucleic acids that encode Phylomers. Phylomer libraries are found to have particular advantages in the practice of the present invention, including in one or more of 3 properties: the high hit-rate; less target bias than other biologics libraries and the potential for high affinity hits which can aid purification of Phylomer/target complexes.

For the purposes of the present description, a "Phylomer" is peptide of about 8 to about 180 amino acids encoded by a nucleic acid fragment obtainable from a genome (or transcriptome) of a micro-organism and/or a genome of a small (such as a compact) genome of a eukaryotic species, in particular a nucleic acid fragment obtainable (or obtained) from a genome of a micro-organism such as a prokaryote. In certain embodiments, the nucleic acid fragment is obtained from such an organism, and also include those obtainable from an organism for which the genome is well-characterised or has been sequenced, and/or is a fragment that is between about 24 to about 550 nucleotide base pairs. For example the fragment may be obtainable from a prokaryotic genome (or transcriptome) and/or from a genome (or transcriptome) of Aeropyrum pernix, Aquifex aeolicus, Archaeoglobus fulgidis, Bacillus subtilis, Bordetella pertussis, Borrelia burgdorferi, Chlamydia trachomatis, Escherichia coli, Haemophilus influenzae, Helicobacter pylori, Methanobacterium thermoautotrophicum, Methanococcus jannaschii, Mycoplasma pneumoniae, Neisseria meningitidis, Pseudomonas aeruginosa, Pyrococcus horikoshii, Synechocystis PCC 6803, Thermoplasma volcanium and Thermotoga maritima. Sources of nucleic acid fragments that encode Phylomers include nucleic acids from Fugu rubripes, Caenorhabditis elegans, Saccharomyces cerevisiae, Escherichia coli, Aquifex aeliticus, Methanococcus jannaschii, Bacillus subtilis, Haemophilus influenzae, Helicobacter pylori, Neisseria meningiditus, Synechocystis sp., Bordetella pertussis, Pasteurella multocida, Pseudomonas aeruginosa, Borrelia burgdorferi, Menthbacterium thermoautotrophicum, Mycoplasma pneumoniae, Archaeoglobus fulgidis, or Vibrio harveyi, or from any species described in TABLE 1. The nucleic acid fragments may be obtained and/or generated using art-recognised methods e.g., mechanical shearing, digestion with a nuclease, digestion with a restriction endonuclease, amplification by polymerase chain reaction (PCR) using random oligonucleotide primers, and combinations thereof.

A Phylomer may be between about 10 and about 165, such as between about 15 and 120 amino acids in length, including being about, 20, 30, 40, 50, 60, 70, 80, 90, 100, or 110 amino acids in length, and/or may be encoded by a nucleic acid fragment obtainable from (eg from a genome or transcriptome of) a micro-organism or small (such as a compact) genome of a eukaryotic species that has a length corresponding to that encoding an amino acid of any of such lengths.

In certain embodiments, the Phylomer peptide has a biological activity, for example a biological activity that is different from any activity the peptide has in its native environment, if any. For example, a Phylomer may bind a target protein in the mammalian cell, such as a mammalian protein, and/or does not bind such target protein if expressed within the organism from which the nucleic acid encoding such Phylomer is obtainable. For example, the target protein may not be found in nature within such organism, the Phylomer may not be expressed in such organism, or only as part of a larger protein that has a different function.

A Phylomer library is a population of Phylomers having diverse sequences. For example, a Phylomer library may comprise (or may be expressed from a library of nucleic acids encoding) 1 x 10³ or more, about 3 x 10³ or more, 3 x 10⁴ or more, 1 x 10⁵ or more, 1 x 10⁶ or more, 1 x 10⁷ or more, 1 x 10⁸ or more different Phylomer sequences, preferably 1 x 10⁸ to 1 x 10⁹' preferably between 1 x 10⁹ and 1 x 10¹⁰ or more different Phylomer sequences; preferably between 1 x 10¹⁰ and 1 x 10¹¹ or more different Phylomer sequence, preferably between 1 x 10¹¹ and 1 x 10¹² or more different Phylomer sequence, preferably between 1 x 10¹² and 1 x 10¹³ or more different Phylomer sequence. In particular embodiments: (i) the library of Phylomers comprises 3 x 10⁴ or more, such as 1 x 10⁶ or more, different amino acid sequences; (ii) or said library of Phylomers is expressed from a plurality of nucleic acids comprising 3 x 10⁴ or more, such as 1 x 10⁶ or more, different nucleic acid sequences that encode Phylomers. Also, (a) the library of Phylomers may comprise 3 x 10⁴ or more, such as 1 x 10⁶ or more, different Phylomers; or (b) the library of Phylomers is expressed from a plurality of nucleic acids that may encode 3 x 10⁴ or more, such as 1 x 10⁶ or more, different Phylomers.

Libraries of Phylomers may be generated from nucleic fragments obtained from two or more of the microorganisms or eukaryotic species having a small (compact) genome, such as (but not limited to) two or more of any such organisms described herein. In certain embodiments, a Phylomer libraries is generated from nucleic fragments obtained from three or more such organisms, such as between about five and about 100 such organisms. For example, a Phylomer library may be obtained from about 6, 10, 15, 20, 25, 30, 35, 50, 60. 70, 80 or 90 such organisms. One suitable Phylomer library may be obtained from nucleic acid fragments obtained from at least 5 or the organisms listed in TABLE 1. In particular embodiments of the libraries, the organisms from which the nucleic acid fragments are obtained are selected from such organisms which are evolutionary diverse. For example, no more than 1, 2, 3, 4 or 5 organisms from any given region(s) of the phylogenetic tree are used for the generation of a Phylomer library.

Phylomer libraries may be constructed using any convenient technique. For example, a Phylomer library may be constructed by randomly cloning short fragments of nucleotide sequence from one or more microbial nucleic acids into expression vectors. A Phylomer library may be produced by a method comprising: (i) producing fragments from nucleic acids from two or more organsisms described herein; (ii) inserting the nucleic acid fragments into an expression vector adapted to express the fragment; and (iii) expressing the peptide encoded by the nucleic acid fragment.

As described herein, the nucleic acid fragments may be produced from a mixture of nucleic acids (i.e. genomes or transcriptomes) from different of such organisms. The nucleic acids may be present in the mixture in an amount that is proportional to the complexity and size of the genome (or transcriptome), for example, in comparison to the complexity and size of other genomes in the mixture. This results in an approximately equal representation of the genome (or transcriptome) fragments from the respective organisms.

Nucleic acid fragments may be generated from one, two or more genomes (or transcriptomes) of the subject organsisms by one or more of a variety of methods known to those skilled in the art. Suitable methods include, as well as those described in the examples below, for example, mechanical shearing (e.g. by sonication or passing the nucleic acid through a fine gauge needle), digestion with a nuclease (e.g. Dnase 1), partial or complete digestion with one or more restriction enzymes, preferably frequent cutting enzymes that recognize 4-base restriction enzyme sites and treating the DNA samples with radiation (e.g. gamma radiation or ultra-violet radiation). In some embodiments, nucleic acid fragments may be generated from one, two or more the subject organisms by low temperature primer extension or by polymerase chain reaction (PCR) using, for example, random or degenerate oligonucleotides. Random or degenerate oligonucleotides may include restriction enzyme recognition sequences to allow for cloning of the amplified nucleic acid into an appropriate nucleic acid vector.

Each fragment of nucleic acid obtained as described above encodes a Phylomer. The fragments may be cloned into expression vectors for expression of the Phylomer as a peptide.

Nucleic acid encoding a Phylomer may be flanked (for example 5' and 3' to the coding sequence) by specific sequence tags. Sequence tags comprise 10 to 50 nucleotides of known sequence which may be used as binding sites for oligonucleotide primers. Preferably, the sequence of the tag is not found in the mammalian genome. This allows the coding sequence of a Phylomer to be conveniently amplified from the mammalian cell, for example by PCR, as required.

Nucleic acid encoding the Phylomer may be operably linked to a regulatory element. Suitable regulatory elements and vectors are well known in the art, and include those described elsewhere herein. Suitable techniques for producing and manipulating nucleic acid and expressing it in mammalian cells are well known in the art by the person of ordinary skill.

Nucleic acid encoding the Phylomer may be operably linked to an element controlling translation such as Kozak sequences, Internal Ribosome Entry Sequences (IRES elements), and/or to elements promoting translational 'slippage'. Suitable regulatory elements and vectors are well known in the art, and include those described elsewhere herein. Suitable techniques for producing and manipulating nucleic acid and expressing it in mammalian cells are well known in the art by the person of ordinary skill.

Nucleic acid encoding Phylomers as described herein may be readily prepared, manipulated, cloned and expressed by the skilled person using standard techniques (for example, see Molecular Cloning: a Laboratory Manual: 3rd edition, Sambrook and Russell (2001) Cold Spring Harbor Laboratory Press; Molecular Biology, Second Edition, Ausubel et al. eds. John Wiley & Sons, 1992).

Phylomers and Phylomer libraries are known in the art (Watt et al (2006) Nat Biotech 24 17-183; Watt et al (2006) Expert Opin Drug Disc 1 491-502, Watt et al (2009) Future Med Chem 1 (2) 257-265, WO 2000/041967, WO 2000/068373, WO 2005/119244; WO 2004/074479 and WO 2006/017913).

A Phylomer library may be employed as described herein in a form represented by libraries of nucleic acids that encode the plurality of Phylomers. For example, in some embodiments, nucleic acid encoding a Phylomer library may be contained in plasmids suitable for expression in mammalian cells. The plasmids may be transfected or virally transduced into a population of mammalian cells and the Phylomer sequences expressed. Such expression by and within the mammalian cells thereby exposes the mammalian cell to the Phylomer, and when conducted on a library scale to a population of mammalian cells thereby exposes a population of such cells to a library of Phylomers. As will be known to the person of ordinary skill, by the inclusion of suitable localisation or secretion tags or sequences, the Phylomers expressed from the nucleic acids may be targeted to particular locations or organelles of the mammalian cell to facilitate modulation of certain phenotypes that involve target proteins so located. For example, target proteins being extracellular receptors may be address by the inclusion of a secretion tag to be co-expressed (as a fusion) with the Phylomer. Suitable methods for the transfection or transduction of mammalian cells with libraries of expression plasmids encoding Phylomers are known to the person of ordinary skill.

In another embodiment, the Phylomers may be produced through cell free expression (eg. By ribosome dispay or CIS-display, as described below). In yet another preferred embodiment, the Phylomer peptides may be produced by synthetic techniques

Alternatively, a Phylomer library may be directly employed as described herein in the form of peptides. For example, a plurality of individual nucleic acids that encode Phylomers may be expressed (for example by yeast or bacterial expression systems or by secretion by insect or mammalian cells into tissue-culture media), the Phylomer peptides so produced collected (and optionally purified), and then pools of Phylomer peptides brought into contact with suitable mammalian cells. Standard de-convolution approaches to identify individual Phylomers (hits/positives) from such a pool of Phylomers can then be used. Furthermore, with suitable high-throughput protein expression and purification methodologies (as are now known in the art), individual Phylomers may be so produced and a plurality of Phylomers so produced may be individually contacted with the mammalian cells. Accordingly, by such methods thousands or more, such as about 10s or 100s of thousands, millions or more, of Phylomer peptides (ie, a library of Phylomers) may be exposed to a population mammalian cells. Suitable transfection methods to aid the contact of peptides with (or penetration into) mammalian cells will be known by the person or ordinary skill. Yet, in certain of such embodiments, such as if intracellular protein targets are to be investigated, the Phylomer peptides to be contacted with the mammalian cells may further comprise a cell-penetration signal or moiety to aid the penetration of the Phylomer peptide into the mammalian cell. Such a moiety may comprise cell-penetration peptide sequence such as TAT, TAT-like or other cell-penetrating peptides (CPP) sequences as are well known in the art and may be derived from Phylomer libraries. Such CPP-Phylomer fusion can be readily produced by appropriate design of the expression system. CPPs may be useful in transporting a Phylomer peptide into a cell, for example to screen for effects on cell phenotype directly with Phylomer peptides, such as described herein.

A CPP is a heterologous amino acid sequence that facilitates transport of an attached moiety across a cell membrane. Suitable CPPs are well-known in the art including, basic peptides, such as Drosophila homeoprotein antennapedia transcription protein (AntHD), HSV structural protein VP22, HIV TAT protein, Kaposi FGF signal sequence (kFGF), protein transduction domain-4 (PTD4), Penetratin, M918, Transportan-l0, PEP-I peptide, nuclear localization sequences, amphipathic peptides, and peptide sequences comprising 5 or more contiguous basis residues, such as arginines or lysines (e.g. (R)9, (K)9, (R)11, or (K)11). Other suitable CPPs are known in the art (see for example Inoue et al., 2006 Eur. Urol. 49, 161-168; Michiue et al., 2005 J. Biol. Chem. 280, 8285-8289; Wadia and Dowdy, 2002 Curr. Opin. Biotechnol. 13 52-56; Langel (2002) Cell Penetrating Peptides, CRC Press, Pharmacology and Toxicology Series; US6730293, WO05/084158 and WO07/123667)). Wadia & Dowdy Current Opin Biotechnology (2002) 13 52-56; Wagstaff & Jans Curr Medicinal Chemistry 13 1371-1387 (2006). Other CPPs, including those derived from Phylomer libraries are describe in co pending applications AU 2011901997 and US 61/489,198.

Following exposure of the Phylomer library to the population of mammalian cells, the population of cells may be screened or otherwise investigated for the presence, absence, alteration or other modulation of the phenotype, such as the phenotypic trait or characteristic.

As described above, the population may be screened for the appearance or enhancement of a phenotypic trait or characteristic which the cells used as described herein do not normally display. For example, the cells may be cells with a disease phenotype, such as cancer cells, and may be screened for the appearance (or enhancement) of a phenotypic trait or characteristic which is characteristic of normal cells. As a further example, the expression of a marker on the surface of the mammalian cell may be an appearance of a phenotype that is screened for, such as by using a fluorescently labelled antibody that binds to such marker and fluorescence-activated cell sorting (FACS).

The population may be screened for the disappearance or reduction of a phenotypic trait or characteristic which is displayed by the cells used as described herein. For example, the cells may be cells with a disease phenotype, such as cancer cells, and may be screened for the disappearance (or reduction) of a phenotypic trait or characteristic which is characteristic of the disease. The phenotypic trait or characteristic may be associated with inhibition of a cell signalling pathway, for example a cell signalling pathway which is active in cancer cells.

The population may be screened for a detectable change in the degree of a phenotypic trait or characteristic, such as an increase or decrease in a quantitative phenotype. For example, the signal generated from a recombinant reporter gene, such as a luminescent or fluorescent protein, may be quantitatively determined and cells in the population that display an alteration or modulation of the phenotype identified by a change in the fluorescent or light intensity of the given cell, or cell culture of a clone of such cells. In particular embodiments, the change in the phenotype to be screened is a reduction (or an increase) in luminescence or fluorescence associated with the mammalian cell, for example associated with a recombinant reporter gene (or surface marker) in said cell.

One or more cells in the population which display an altered phenotype after Phylomer exposure are identified. Cells with altered phenotypes may be identified by any convenient method. For example, a high content screening platform such as the Cellomics ArrayScanTM may be used to screen a Phylomer library, either in plasmid library or synthesised peptide form, for Phylomers which alter or otherwise modulate cell phenotypes.

Alternatively, cells with altered phenotypes may be identified through alterations in cell surface marker expression, for example, using fluorescence-activated cell sorting (FACS), or through expression of phenotype-associated enzymes, such as β-galactosidase, for example using biochemical assays. In a particular embodiment, the phenotype is luminescence or fluorescence signal generated by a reporter gene, and cells that display an altered phenotype are identified via a change (eg an increase or decrease) of luminescent or fluorescent signal using techniques and equipment known to the person of ordinary skill. For example, plate-readers, CCD detectors and scanning apparatus may be employed to identify a cell in the population that displays an alteration in a luminescent or fluorescent phenotype.

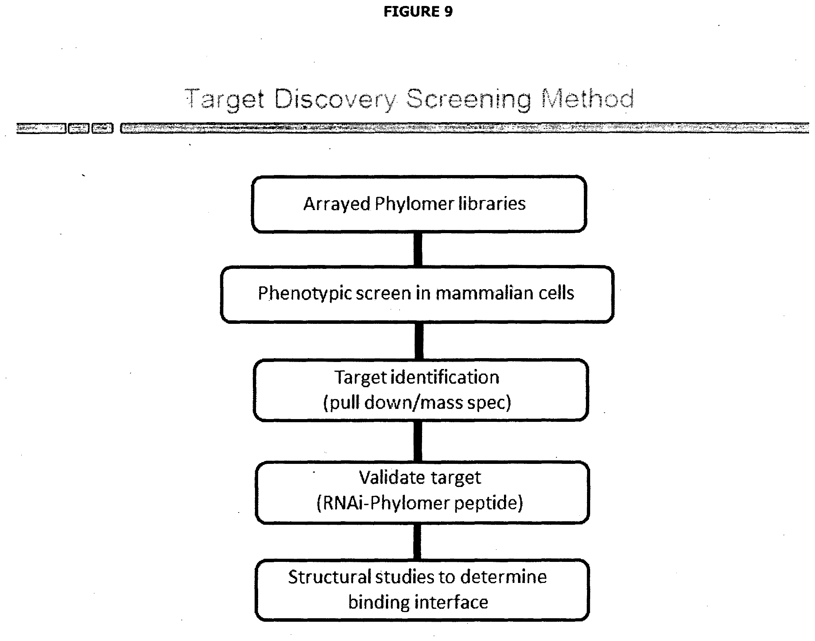

In particular embodiments of the methods described herein (i) the library of Phylomers comprises a plurality of separate and addressable Phylomers, optionally fused to cell penetrating peptide sequences; or (ii) the library of Phylomers is expressed from a plurality of separate and addressable nucleic acids that encode Phylomers. By "separate and addressable" includes a plurality, of individual Phylomers (or nucleic acids that encode such Phylomers) - such as 1 x 10³ or more, about 3 x 10³ or more, 3 x 10⁴ or more, 1 x 10⁵ or more, 1 x 10⁶ or more, 1 x 107 or more, 1 x 10⁸ or more individual moieties - that are ordered and/or identified in such as way that an individual moiety can be recovered, deconvoluted and/or identified. For example, (i) the plurality of separate and addressable Phylomers are exposed to said population of mammalian cells arranged in an array-format; or (ii) the plurality of separate and addressable nucleic acids are expressed in said population of mammalian cells arranged in an array-format. Array-format includes where the plurality or the respective moieties are arranged in a regular spatial arrangement or pattern, such as in racked-tubes or microtitre plate-based arrangements. The person of ordinary skill will be aware of suitable 48-well, 96-well, 384 and higher numbers of wells in microtitre plates that may be employed for an array-format method described herein. Other array-formats include spotted or other microarrays of moieties, such as arrays or microarrays of peptides, nucleic acids or cells. FIGURE 9 depicts one possible embodiment of such aspect using arrayed Phylomer libraries.

In other particular embodiments of the methods described herein, the said cell which displays an alteration in said phenotype following said exposure or said expression is identified from said population of mammalian cells arranged in an array-format, such as one described herein. For example, the identification step of the present example may be conducted by analysing microtitre plates of cell cultures - each culture exposed to different Phylomer - for an increase or decrease in luminescent or fluorescent intensity using a plate reader.

As described above, the library of Phylomers (or the plurality of nucleic acids that encode said library) may be comprise a pool, and this pool is employed to expose a population of mammalian cells to the library of Phylomers. Accordingly, in certain embodiments: (i) the library of Phylomers comprises a pooled plurality of Phylomers, optionally fused to cell penetrating peptide sequences; or (ii) the library of Phylomers is expressed from a pooled plurality of nucleic acids that encode Phylomers. For certain of such embodiments, it is envisioned that: (i) said Phylomers are exposed to said population of mammalian cells arranged in a pooled-format; or (ii) said plurality of pooled nucleic acids are expressed in said population of mammalian cells arranged in a pooled-format.

In particular embodiments of the methods described herein, for example when the cell population is exposed to a pooled Phylomer library, a cell which displays an alteration in said phenotype following said exposure (or expression of nucleic acid encoding said library of Phylomers) is identified using fluorescence-activated cell sorting (FACS).

Cells identified as displaying an altered phenotype may be isolated and/or purified. Accordingly, the methods described herein include embodiments that comprise isolating, from the population of mammalian cells, at least one cell which displays an alteration in phenotype following exposure to the library of Phylomers

Cells displaying an altered phenotype may be isolated by any suitable technique. For example FACS may be employed. Alternatively, a cell may be sampled or aliquoted and further cultured. Accordingly, in some embodiments, the one or more cells may be cultured and/or expanded to produce one or more populations of cells that are capable of displaying (or display) the altered phenotype.

The cell (such as the isolated cell or cell culture) is employed to identify the Phylomer that leads to, causes or otherwise is otherwise associated with the alteration in the phenotype.

In one embodiment: (i) a Phylomer is isolated and/or identified from said isolated cell; or (ii) a nucleic acid encoding a Phylomer is isolated by amplifying and/or cloning said nucleic acid from said isolated cell. In case (i), technologies such as affinity capture or purification and/or protein micro sequences (eg by protease digestion followed by mass-spectrometric analysis, or by protein micro-array analysis) may be employed to so isolate and/or identify the Phylomer peptide from the isolated cell, such as by isolation and sequencing. In case (ii), the isolated nucleic acid encoding a Phylomer may be identified by sequencing. Alternatively such nucleic acids can be identified by means of their association with a 'bar-code' sequence or other such (molecular) identification tag.

In another embodiment, for example when employing separate and addressable Phylomer libraries or nucleic acids encoding such libraries, the Phylomer that leads to, causes or otherwise is associated with the alteration in the phenotype is identified by reference to the respective address. Referencing back to the source or original address of the Phylomer can directly provide the identity (such as the amino acid sequence) of the Phylomer if, for example, the sequences of the Phylomer within the library are already known. Alternatively a sample of the Phylomer peptide at the source address (or the nucleic acid therein) may be sampled and sequenced in order to identify the amino acid sequence of the Phylomer that leads to, causes or otherwise is otherwise associated with the alteration in the phenotype.

The nucleic acids encoding Phylomers that lead to, cause or otherwise are otherwise associated with the alteration in the phenotype in a mammalian cell (such as those expressed in the one or more cells identified as displaying an altered phenotype) may be isolated. Any convenient technique may be employed. For example, total nucleic acid may be extracted from the cells (or from the original source-address for an addressable library) and the nucleic acids encoding the Phylomers amplified or cloned therefrom. In some particular embodiments, the nucleic acid may be amplified using primers which hybridise to the sequence specific tags flanking the Phylomer coding sequence. After isolation, nucleic acids encoding the Phylomers may be further amplified, sequenced, re-cloned into new vectors and/or otherwise manipulated. In other preferred embodiments, nucleic acids encoding active Phylomers may be amplified directly from the cellular environment in which the alteration of phenotype was observed and then identified through nucleic acid sequencing.

In some embodiments, a population of Phylomers identified from the cells identified as displaying an altered phenotype may be subjected to one, two, three or more additional rounds of phenotypic screening as described above.

The Phylomer identified as leading to, causing or being otherwise associated with the alteration in the phenotype is provided for subsequent steps of the methods. Various approaches for the production of Phylomers are available. Encoding nucleic acid may be expressed to produce the Phylomer (see for example, Recombinant Gene Expression Protocols Ed RS Tuan (Mar 1997) Humana Press Inc). Alternatively, Phylomers may be generated wholly or partly by chemical synthesis. Phylomers may be synthesised using liquid or solid-phase synthesis methods; in solution; or by any combination of solid-phase, liquid phase and solution chemistry, e.g. by first completing the respective peptide portion and then, if desired and appropriate, after removal of any protecting groups being present, by introduction of the residue X by reaction of the respective carbonic or sulfonic acid or a reactive derivative thereof. Chemical synthesis of peptides is well-known in the art (J.M. Stewart and J.D. Young, Solid Phase Peptide Synthesis, 2nd edition, Pierce Chemical Company, Rockford, Illinois (1984); M. Bodanzsky and A. Bodanzsky, The Practice of Peptide Synthesis, Springer Verlag, New York (1984); J. H. Jones, The Chemical Synthesis of Peptides. Oxford University Press, Oxford 1991; in Applied Biosystems 430A Users Manual, ABI Inc., Foster City, California; G. A. Grant, (Ed.) Synthetic Peptides, A User's Guide. W. H. Freeman & Co., New York 1992, E. Atherton and R.C. Sheppard, Solid Phase Peptide Synthesis, A Practical Approach. IRL Press 1989 and in G.B. Fields, (Ed.) Solid-Phase Peptide Synthesis (Methods in Enzymology Vol. 289). Academic Press, New York and London 1997).

In particular embodiments, the provided (identified) Phylomer is provided as part of a fusion protein that comprises the Phylomer and an affinity tag, or otherwise fused to a heterologous peptide. For example, following identification and isolation of nucleic acid encoding a Phylomer which alters cell phenotype, the nucleic acid may be re-cloned into an expression vector adjacent to nucleic acid encoding a heterologous peptide, such that the vector expresses a fusion protein comprising the Phylomer fused to the heterologous peptide. Suitable heterologous peptides include affinity tags and CPPs (as described above).

An affinity tag is a heterologous amino acid sequence that forms one member of a specific binding pair. Peptides containing an affinity tag may be isolated and/or detected through the binding of the other member of the specific binding pair to the affinity tag. For example, the affinity tag may be an epitope which is bound by an antibody molecule. Suitable affinity tags are well-known in the art including, for example, MRGS(H)6, DYKDDDDK (FLAGTM), T7-, S- (KETAAAKFERQHMDS), poly-Arg (R5-6), poly-His (H2-10), poly-Cys (C4) poly-Phe(Fll) poly-Asp(D5-16), Strept-tag II (WSHPQFEK), c-myc (EQKLISEEDL), Influenza-HA tag (Murray, P. J. et al (1995) Anal Biochem 229, 170-9), Glu-Glu-Phe tag (Stammers, D. K. et al (1991) FEBS Lett 283, 298-302), Tag.100 (Qiagen; 12 aa tag derived from mammalian MAP kinase 2), Cruz tag 09™ (MKAEFRRQESDR, Santa Cruz Biotechnology Inc.) and Cruz tag 22™ (MRDALDRLDRLA, Santa Cruz Biotechnology Inc.). Known tag sequences are reviewed in Terpe (2003) Appl. Microbiol. Biotechnol. 60 523-533.

Affinity tags may be useful in purifying and/or isolating the Phylomer during production, and/or for example for the immunoprecipitation of Phylomers bound to cellular binding partners.

Tandem Affinity Tags (or 'TAP' tags) may be employed as described herein, such as in the purifification and/or isolation the Phylomer during its production, and/or for example for the immunoprecipitation of the Phylomer when bound to a cellular binding partner in order to improve yield and reduce background.

Having identified a Phylomer that alters a phenotype of a mammalian cell, and providing the Phylomer, optionally as a fusion protein, a method may further comprise confirming the effect of the Phylomer on the phenotype of a mammalian cell. For example, Phylomer peptides which have been synthesised with a Cell-Penetrating Peptide (CPP) or cargo peptide sequence may be used directly on the cells in order to elicit a phenotypic alteration, thereby confirming the effect of the Phylomer.

In a method described herein, a cellular protein is identified to which the Phylomer that leads to, causes or is otherwise associated with the alteration in phenotype binds. Such a cellular protein is hence considered, eg is, or being, a target protein that modulates the phenotype under investigation as described herein.

A cellular protein to which the Phylomer binds may be identified by various means. In particular, Phylomer peptides may be used to identify the cellular binding partner(s) of such Phylomer. For example, cellular proteins that specifically interact with or bind, and/or with high affinity, to the Phylomer may be identified.

Cellular proteins which bind to the Phylomer may be identified using standard screens for cellular binding partners, such as those described in the examples below. For example, the Phylomer may be used as a bait molecule to identify molecules in a mammalian cell or cell extract.

Cellular proteins which bind to the bait Phylomer may be isolated. Accordingly, the methods described herein may include a step comprising, prior to identification of a cellular protein, isolating a cellular protein which binds to the provided (identified) Phylomer. Suitable techniques are well known in the art and include techniques such as radioimmunoassay, co-immunoprecipitation, two-hybrid techniques, the probing of arrays of candidate proteins using labelled Phylomers, scintillation proximity assays and ELISA methods. For example, the Phylomer may be over-expressed in mammalian cells and immunoprecipitated with antibodies binding to the epitope tag.

Following isolation, cellular proteins bound to the bait Phylomer may be analysed and/or identified. Suitable techniques are well known in the art and include mass spectrometry, for example, MALDI-linked TOF mass spectrometry. In particular embodiments, the cellular protein is isolated by contacting a mammalian cell extract with said provided Phylomer, under conditions permitting the binding of said provided Phylomer and the cellular protein, and isolating a complex comprising said provided Phylomer and the cellular protein bound thereto. Optionally, the complex is isolated by purification; and preferably such purification is effected using an affinity tag within a fusion protein which comprises said provided Phylomer and said affinity tag. Suitable methods to provide a Phylomer-affinity tag fusion are described above. In further embodiments, the cellular protein is identified by mass spectrometry or by protein-microarray analysis with said provided Phylomer.

Methods described herein may also relate to the characterisation of an interaction site on a target protein. Such methods employ Phylomers, which are found to have advantageous properties when employed at such step of the methods. For example, the interaction site of a target protein may be characterised by performing a further Phylomer screen. To perform a further Phylomer screen, the target protein may be provided in a form which is convenient for the method of screening to be employed. Accordingly, the target protein may be provided in isolated form; for example, a protein may be chemically synthesised or expressed recombinantly and purified. Optionally, the isolated target protein may be immobilised. For some screening methods, such as phage display, an isolated target protein may be advantageously immobilised on a substrate, such as a multiwell plate, for screening. In particular embodiments, the target protein is provided from expression of a nucleotide sequence encoding said target protein in a host cell. The person of ordinary skill will be aware of suitable methods to produce the target protein, such as by expression of such a nucleotide sequence.

Suitable target proteins may include target proteins identified by a Phylomer based phenotypic screen as described above and known target proteins.

In other embodiments, a nucleic acid encoding the target protein or fragments thereof, such as known protein:protein interaction domains, may be expressed in host cells in which screening is performed. Suitable expression methods are well-known in the art. The host cell and/or the nucleic acid may be adapted for the screening method which is employed. For example, for a two hybrid screen, a nucleic acid may encode a fusion protein comprising the target protein linked to a heterologous peptide, such as the DNA binding domain or activation domain of transcription factor, as described below.

A library of Phylomers may be screened to identify a population of Phylomers that bind to the target protein. Accordingly, said population of Phylomers is identified by screening a library Phylomers, or of nucleic acids that encode Phylomers, for binding of said encoded Phylomers to said target protein. The library of Phylomers or the library of nucleic acids may be a diverse libraries. That is, it may comprise a number of different sequences such as described above.

The library of Phylomers, which may be represented by a plurality of nucleotide sequences in plasmid form or CPP-Phylomer peptide form, may be screened for binding to the target protein using any convenient technique. Suitable screening methods are well known in the art and include phage or ribosome display and two-hybrid screening, for example in yeast or mammalian cells or *in vitro.* Accordingly, in certain embodiments, the library is screened using a two-hybrid screen, phage display or via in vitro (eg. Ribosome or CIS-) display.

In some embodiments, screening may be performed using a two-hybrid screen. Two hybrid screens typically employ a transcription factor which has a DNA binding domain and a transcriptional activation domain. Suitable transcription factors include GAL4, which has a DNA binding domain (GAL4DBD), and a GAL4 transcriptional activation domain (GAL4TAD) and combinations of DNA binding domains and transcriptional activation domains, such as the LexA DNA binding domain and the VP60 transcriptional activation domain. The two hybrid assay format is well-known in the art (see for example Fields and Song, 1989, Nature 340; 245-246).

In particular embodiments, the SOS-recruitment system (Aronheim et al 1997) is used to identify a population of Phylomers that bind to the target protein. The SOS-Recruitment-System is based on the activation of a mitogenic signaling pathway in the yeast Saccharomyces cerevisiae (S. cerevisiae). Briefly, the recombinant bait target protein is fused to the coding region of truncated hSos1. An expression plasmid, allowing constitutive expression of the bait is co-transformed(Gietz and Schiestl, 2007) with the library expressing the Phylomer library into a cdc25-2 yeast strain. Phylomer peptides may be expressed from an inducible GAL1 promoter as fusions to a lipidation signal for membrane attachment. Interactions of bait and prey proteins can be tested in a yeast strain, whose endogenous RAS pathway is regulated by a temperature sensitive mutation (cdc 25-2). Putative interactors can be shifted to the restrictive temperature (37°C) and tested for galactose dependency to identify the target protein-interacting Phylomers. The peptide coding inserts of these clones can then be isolated and subjected to sequencing.

By fusing a Phylomer to one of those domains, and the target protein or a fragment thereof which comprises the interaction site to the respective counterpart, a functional transcription factor is restored only when the Phylomer binds to the target protein. Thus, binding of a Phylomer to a target protein may be measured by the use of a reporter gene which is operably linked to a binding site for the transcription factor DNA binding domain which is capable of activating transcription of said reporter gene.

Two hybrid screening may be performed in yeast or mammalian cells. Suitable host cells may comprise a heterologous nucleotide sequence encoding a first detectable reporter. A detectable reporter is a polypeptide which can be detected when it is expressed in a cell. For example, expression of the detectable reporter may lead to the production of a signal, such as a fluorescent, bioluminescent or colorimetric signal, which can then be detected using routine techniques. The signal may be produced directly from the reporter, after expression, or indirectly through a secondary molecule, such as a labelled antibody.

Suitable detectable reporters include fluorescent proteins which produce a detectable fluorescent signal. Suitable fluorescent reporters include Cherry and GFP or any other pairs of fluorescent proteins whose excitation/emission spectra are suitably separated to allow them to be used for flow cytometry.

The nucleotide sequence encoding the first detectable reporter may be operably linked to a regulatory element which is activated by a transcription factor. The regulatory element activates transcription of the nucleotide sequence encoding the reporter when the DNA binding domain and the transcriptional activation domain of the transcription factor are brought together at the regulatory element by binding between the Phylomer and the target protein. The detectable reporter is therefore only expressed in cells which express a Phylomer which binds to the target protein.

Suitable host cells may further comprise a heterologous nucleotide sequence encoding the target protein fused to a first domain of the transcription factor (i.e. one of the DNA binding domain and the transcriptional activation domain). The nucleotide sequence may be contained in an expression vector and operably linked to a regulatory element.

Heterologous or exogenous nucleotide sequences encoding the detectable reporter and target protein may be incorporated within the genome of the host cell or contained in extra-chromosomal vectors.

In some embodiments, a reverse yeast-2-hybrid screen (Vidal et al., 1996) may be employed in which binding of a target protein with a binding partner causes the death of a cell. Phylomers which block this binding rescue the yeast cell from dying and so may be readily identified in a screen.

A modification of a reverse yeast two-hybrid system (originally described by Vidal et at al. 1996) allows a second counter-selectable marker (*CYH2*) and stringency titration by adjustment of sugar concentrations in the screening media. Briefly, the target protein (or fragments thereof) may be cloned into yeast two-hybrid vectors pDD [pGilda bait vector modified by replacing the ampicillin selection gene with kanamycin selection] and pJFK [pYesTrp prey vector (Invitrogen), modified by replacing the *TRP1* yeast selection gene with *HISS* and replacing the ampicillin selection gene with kanamycin selection], respectively. Bait and prey constructs can be co-transformed into S. *cerevisiae* strain PRT480 (MATa, his3, trp1, ura3, 4 LexA-LEU2, lys2::3 cIop-LYS2, CAN^{R}, CYH2^{R}, ade2::2 LexA-CYH2-ZEO, his5::2 LexA-URA3-G418) using a lithium-acetate based chemical transformation protocol (Ausubel et al. 1989). The blocking Phylomer peptide library may be transformed into *S*. *cerevisiae* strain PRT51 (MAT-, his3, trp1, ura3, 6 LexA-LEU2, lys2::3 cIop-LYS2, CYH2^{R}, ade2::G418-pZero-ade2, met15::Zeo-pBLUE-met15, his5::hygro), using a modified high-efficiency chemical transformation protocol (Gietz and Schiestl, 2007). Bait/prey plasmid containing PRT480 haploids (10⁸ cells) can be mated with the blocking Phylomer library (10⁷ cfus), and plated to HW⁻ minimal media (minimal media lacking histidine and tryptophan) to select for diploids. After 2 days incubation at 30°C, plates are scraped and harvested yeast cells were washed, resuspended 1:1 (v/v) in yeast freezing solution (65% v/v glycerol, 0.1M MgSO₄, 25 mM Tris-CI pH 8.0), and frozen at -80°C in 1ml aliquots. To select for blockers, 1.5x10⁷ target protein/Phylomer diploids cfus can be thawed and outgrown overnight in HW⁻ to achieve log-phase growth. The following day, 3x10⁵ diploids are plated onto counter-selective media: HWU⁻ (lacking histidine, tryptophan and uracil), containing supplements of 0.02% galactose (gal), 2% raffinose (raff), 0.2 µg/ml uracil, 0.06% (w/v) 5-Fluoroorotic acid (FOA), 5 µg/ml cycloheximide. These plates can be incubated for 7 days, then colonies were picked to HWU⁻ 0.02% gal, 2% raff, and then to HWL⁻ (lacking histidine, tryptophan and leucine) 0.02% gal, 2% raff to confirm blocking phenotype.

To screen the Phylomer library, a population of expression vectors encoding a library of Phylomers fused to a second domain of the transcription factor (i.e. the other of the DNA binding domain and the transcriptional activation domain) is transfected into host cells as described above. If the Phylomer which is expressed in a host cell binds to the target protein, the first and second domains of the transcription factor are brought together, causing the nucleotide sequence encoding the detectable reporter to be transcribed. Expression of the detectable reporter is therefore indicative of binding between the Phylomer and the target protein.

The expression of the detectable reporter may be determined in the transfected host cells in the population. Any suitable approach may be employed to detect expression, depending on the detectable reporter which is used.

Cells which express the detectable reporter may then be isolated and the nucleic acid encoding the Phylomers may be isolated and/or amplified from the cells.

In other embodiments, screening may be performed using phage display techniques. For example, a recombinantly produced library of expressed Phylomers may be screened from Phylomers which bind to the target protein e.g. using lambda bacteriophage or filamentous bacteriophage which display functional Phylomers on their surfaces; for instance see WO 92/01047. Suitable phage display techniques are well known in the art. Typically, a population of phage particles displaying a library of Phylomers is contacted with immobilised target protein. Phage particles which bind to the immobilised target protein may then be purified and/or isolated from the rest of the population. In some embodiments, multiple rounds of phage display may be employed.

A population of phage particles displaying Phylomers which bind to the immobilised target protein may be isolated. Nucleic acid encoding Phylomers which bind to the immobilised target protein may be isolated and/or amplified from the isolated phage particles and manipulated sequenced re-cloned and/or expressed.

In yet other embodiments the Phylomer library (or the plurality of nucleinc acids encoding such library) is screened using *in-vitro* display, such as described by Odegrip, (2004, PNAS, 101: 2806-2810)

In yet other embodiments the Phylomer library (or the plurality of nucleinc acids encoding such library) is screened using yeast display, such as described by Rakestraw, et al. (2011, Protein Engineering Design and Selection, 24: 525-530)

Following identification of the population of Phylomers that bind to the target protein (in particular at the interaction site), in certain embodiments a method described herein comprises testing at least one of the Phylomers provided within the population of Phylomers for its ability to modulate said phenotype of a mammalian cell capable of displaying said phenotype; preferably wherein said testing comprises exposing a mammalian cell capable of displaying said phenotype to said Phylomer and determining if said phenotype of said mammalian cell is modulated. Such steps can act as a confirmation that the association between target, Phylomer and/or phenotype is consistent and/or maintained through the various steps of the method. For example, at least one of the Phylomers provided within said population of Phylomers, when exposed to a mammalian cell, may modulate the phenotype in a mammalian cell capable of displaying the phenotype. Such confirmation step(s) may be conducted by exposing the mammalian cell to at least one Phylomer peptide of the population and investigation of the alteration of phenotype. Alternatively, a nucleic acid encoding a Phylomer from the population may be expressed in the mammalian cell and the alteration in phenotype investigated.

In certain embodiments, in a method described herein, the population of Phylomers which bind to the target protein (in particular at the interaction site) comprises at least 5 Phylomers, for example the population comprises about at least 8, at least 10, at least 20, at least 30, at least 40, at least 50, at least 75, or at least 100 Phylomers, such as those from the population which bind to the target protein as may be identified using a screen described herein. In related embodiments, a method described herein comprised the step of identifying a sub-population of Phylomers within said population of Phylomers, where said sub-population comprises Phylomers of different sequences, such as about at least 5, at least 8, at least 10, at least 20, at least 30, at least 40, at least 50, at least 75, or at least 100 different sequences, that bind to the target protein (in particular at the interaction site).

The Phylomers comprised in the population may be: (i) expressed in or contacted with mammalian cells to confirm that they elicit the same phenotypic effect as the original hit Phylomer; (ii) isolated, re-cloned and/or expressed or otherwise produced; (iii) subjected to alanine-scanning mutagenesis, deletion of certain amino acids, or mutagenesis of specific residues, to characterise binding; and/or (iv) produced by recombinant expression from encoding nucleic acid, or by chemical synthesis as described above.

The binding of each of the Phylomers in the population to the target protein (in particular at the interaction site) may be analysed, for example to measure one or more biophysical parameters. Suitable biophysical techniques for measuring binding include surface plasmon resonance (SPR), differential layer interferometry (DLI) and isothermal titration calorimetry (ITC).

In particular embodiments, the thermodynamics of binding of a Phylomer to the target protein (in particular at the interaction site) may be measured and the binding affinity or Kd may be determined. Accordingly, the present description includes embodiments that comprise measuring the binding affinity (or other quantitative measures of binding) of the Phylomer to the target protein for at least one of the Phylomers in the population of Phylomers that are identified as capable of binding to the target protein (in particular at the interaction site). In certain of such embodiments, the binding affinity (or other quantitative measure of binding) is determined for about at least 3, at least 5, at least 8, at least 10, at least 20, at least 30, at least 40, at least 50, at least 75, or at least 100 Phylomers, such as those from the population which bind to the target protein (in particular at the interaction site).

Those Phylomers of the population whose binding (affinity) to the target protein (in particular at the interaction site) is quantitated may be ranked, selected or otherwise classified. For example, following such quantitative assessment of binding, certain embodiments include the identification of a sub-population of Phylomers within the population of Phylomers that bind to the target protein (in particular at the interaction site) with high affinity, such as identifying a Phylomer which binds to the target protein with about a Kd of 500uM or less, 250uM or less, 150uM or less, 100uM or less, 50uM or less, 25uM or less, 10uM or less, 1uM or less, 500nM or less, 250nM or less, 150nM or less, 100nM or less, 50nM or less, 10nM or less or 1nM or less. In certain of such embodiments, the sub-population of Phylomers comprises about at least 2, at least 3, at least 5, at least 8, at least 10, at least 20, at least 30, at least 40 or at least 50 Phylomers, such as Phylomers that bind the target protein (in particular at the interaction site) with high affinity.

In particular embodiments of methods described herein, Phylomers comprised in the population that bind to the target protein are investigated for binding to the target protein at the interaction site. For example, Phylomers from the population that bind the target protein are classified or selected for the property of binding to the interaction site, such as by using competition displacement and/or binding assays, based as displacement and/or binding assays defined herein including fluoresce-polarisation, and using for example, a Phylomer that is known to bind to the interaction site as one of the competitive components in such an assay. It is a particular feature from the description herein that Phylomer libraries are found to be highly suitable (eg because of their the structural diversity and/or scale; including a manageable scale) for providing a plurality of Phylomers, such as more than about 2, 4, 5, 10 or 20 Phylomers, that bind to the target protein at the interaction site. It is of particular advantage in the characterisation of an interaction site to have such a plurality of Phylomers that bind to the interaction site, especially where such Phylomers have different or diverse sequences. Phylomers with different sequences will posses different side chains and functional groups that will interaction (to different degrees or not interact) with the target protein at the interaction site, from which an increased amount and value of binding and other structural information can be obtained. This increased information can, for example, lead to more efficient and effective identification of ligands that bind the target protein at the interaction site, and hence modulate the phenotypic effect, such as the identification of small molecule ligands for pharmacological applications. The determination of the binding affinities of a number of Phylomers in the population, having different sequences, which bind to the interaction site of the target protein allows the identification of elements of the amino acid sequence which affect the binding affinity. Accordingly, a sub-population of Phylomers which bind to the target protein (in particular at the interaction site) may be identified based wholly or partially on their binding affinity and/or other biophysical parameters. For example, a sub-population may consist of Phylomers which bind the target protein, in particular at the interaction site, with a Kd of 250uM or less (such as with any affinity of less than those given above) and which display increased or the most sequence diversity within the population.

The population of Phylomers identified as capable of binding to the target protein may be classified based on other (either alterative or additional) characteristics. For example, specificity of binding to the target protein (in particular at the interaction site), solubility of the Phylomer, length or their ease of production or availability.

In a method described herein, the orientation, configuration or pose of the Phylomer when bound to the target protein (in particular at the interaction site) is empirically determined. That is, experimental data is collected that is employed in the determination of such orientation, configuration or pose. For this determination, it is considered that the employment of in-silico binding, fitting or docking approaches is not considered "empirical", as in such techniques actual experimental data is not collected. Suitable techniques that provide experimental data to be employed for the empirical determination of the binding configuration (orientation or pose) of at least one Phylomer (such as those within the population, or otherwise identified by a method described herein) to the target protein are well known and include X-ray crystallography and nuclear magnetic resonance (NMR). Accordingly, the binding configuration of at least one Phylomers of the sub-population to said target protein (in particular at the interaction site) is empirically determined, such as about at least 2, at least 3, at least 5, at least 8, at least 10 such binding configurations.

For example, the binding configuration of the Phylomer to the target protein (in particular at the interaction site) may be determined by co-crystallising the target protein with its binding (cognate) Phylomer, or soaking it into an appropriate crystal form, and then using X-Ray Crystallography to solve the structure of the bound Phylomer protein complex, thereby determining the binding configuration of the Phylomer to the target protein (in particular at the interaction site). In some embodiments, NMR may be used to define interacting amino acid residues in the protein and the Phylomer.

The binding configuration of a Phylomer is its preferred (i.e. most energetically favourable) (spatial) orientation relative to the target protein, when the Phylomer and target are bound in a stable complex and represent a definition in terms of interacting atomic groups, bond lengths and bond angles of how a Phylomer binds to the target protein (in particular at the interaction site).

The interaction site on the target protein is then characterised from at least one of the binding configurations, such as that of at one of the Phylomers in the sub-population. For example, the combined binding poses of the Phylomer population may define the three dimensional structure of the interaction site and the structural requirements for ligand binding at the site.

Computer- and analytically-aided inspection of the empirically determined binding configurations enables identification of binding interactions between specific residues or positions on the Phylomer(s) and amino-acid residues of the interaction site of the target protein. In particular embodiments, a plurality of binding configurations, such as about 2, 3, 5, or 10 binding configurations are analysed/inspected leading to the identification of common or consensus locations of interaction. The identification of such locations ("hot spots") of binding interaction (energy) provides one approach for the characterisation of the interaction site. Finding such hotspots, given an empirically determined binding configuration, will be obvious to one skilled in the art. For example, placement of a hydrophobic Phylomer amino acid side chain into a hydrophobic pocket on the target protein surface, or placement of a charged Phylomer amino acid side chain in proximity to a charge of opposite polarity within the target protein.

Further or alternative characterisation of the interaction site may be conducted by analysis, inspection or determination of the three-dimensional structure of the interaction site, and/or location of limited or low interaction binding energy. For example, the location within the interaction site not directly involved with binding of the Phylomer may be employed to find regions that may permit side-chain variation (such as to increase solubility of a ligand) without materially affecting its binding to the target protein.

In another and/or additional approach to characterise the interaction site, the orientation (and/or identity) of at least one side chain (for example, a functional group) of said Phylomer is identified that interacts with said protein target. A functional group may for example, be comprised on a positively, negatively, uncharged or hydrophobic side chain of an amino acid that is comprised in the Phylomer, and optionally one that may be amenable to chemical modifications, such as an amino group on a lysine side chain. Analysis of the binding configuration is employed to identify such orientation, and optionally the identification of, the interacting side-chain/functional group. In this manner, the spatial orientation of the effective pharmacophore that represents the binding of the Phylomer to the target protein may be established. In particular of such embodiments, the orientation (and/or identity) of 2, 3, 5, 8 or 10 of such side-chain/functional groups is identified. Such plurality may be identified for a single binding configuration, or may arise from analysis of a plurality, such as of about 2, 3, 5 or 10, binding configurations.

In a further embodiment of methods described herein, the interaction site is further characterised by characterising the three dimensional structure of the interaction site by analysis of said binding configuration; preferably wherein said three dimensional structure is characterised using *in silico* methods.

In particular embodiments, the characterisation of the interactions site employs the analysis of the empirically determined binding configuration together with analysis of biophysical or biological data on the binding Phylomer. Such biophysical or biological data includes any of that described herein, such as binding affinity or degree of affect on phenotype. In this way, a structure/function (of structure activity) relationship may be established for the interaction site and/or the Phylomer in relation to its binding to the target protein. For example, structural characterisation of a range of different Phylomers (with different sequences) binding to the interaction site, combined with biophysical evaluation of Phylomer/target binding affinities provides information on which chemical groups are required at which points to generate binding affinity. This provides further characterisation of the interaction site, and hence has particular utility in the identification of the structure of small molecules which mimic the binding configuration of the Phylomer and to generate a structure/activity relationship from which to base a drug discovery program.

Molecular sites, such as atoms or atomic groups, on the target protein (in particular at or around the interaction site) which interact with population of Phylomers may be identified from the combined binding poses of the sub-population of Phylomers. The identity and spatial coordinates of each of the plurality of molecular sites may be mapped to define the three dimensional structure of the interaction site. The optimal bond angles and bond lengths for binding to these molecular sites may be determined.

Molecular sites, such as atoms or atomic groups, in the Phylomers which interact with the interaction site of the target protein may be identified from the at least one (or aggregate or consensus) binding configuration of a Phylomer, such as one in the sub-population of binding Phylomers. The identity, orientation and/or spatial coordinates of each of the plurality of molecular sites, and the lengths and angles of bonds with the target protein, may be used to map the structural requirements for ligand binding at the interaction site.

For example, a pharmacophore map may be developed. A pharmacophore map defines the identity and the position of chemical groups required by a ligand in order in order to occupy (eg bind to) the interaction site.

In some embodiments, a method may comprise identifying one or more variants of the binding Phylomers, for example, sequences which differ by 1, 2, 3, 4, 5, 6, 7, 8, 9 or 10 amino acids from the sequence of a (orginal or "base") Phylomer in the sub-population, and predicting and/or empirically determining the binding of the one or more such variants to the interaction site. Variant sequences may be produced using standard techniques and binding determined, including as described herein.

The three-dimensional structure of the interaction site may be modelled *in silico.* The structure may comprise the molecular sites on the target protein shown to interact with the Phylomers. Suitable *in silico* modelling packages are available in the art, such as the Schrodinger environment (Schrodinger Inc, USA) and others as described herein.

A structure of a ligand may be fitted or docked to the characterised interaction site, such as analysed for its dockability to the interaction site. A ligand may be a peptide (such as one having the size and/or other characteristics as described for Phylomers), or may also be a small molecule. A small molecule includes any organic or inorganic chemical molecule that has a molecule weight of less than about 800 Dalton (Da), such as about less than 500 Da, less than 450 Da, less than 400 Da, less than 350 Da, less than 300 Da or less than 250 Da. The small molecule may have biophysical characteristics that make it suitable for a biological application, including that of solubility, lack of toxic groups and/or other characteristics such as Lipinski's rule of five.

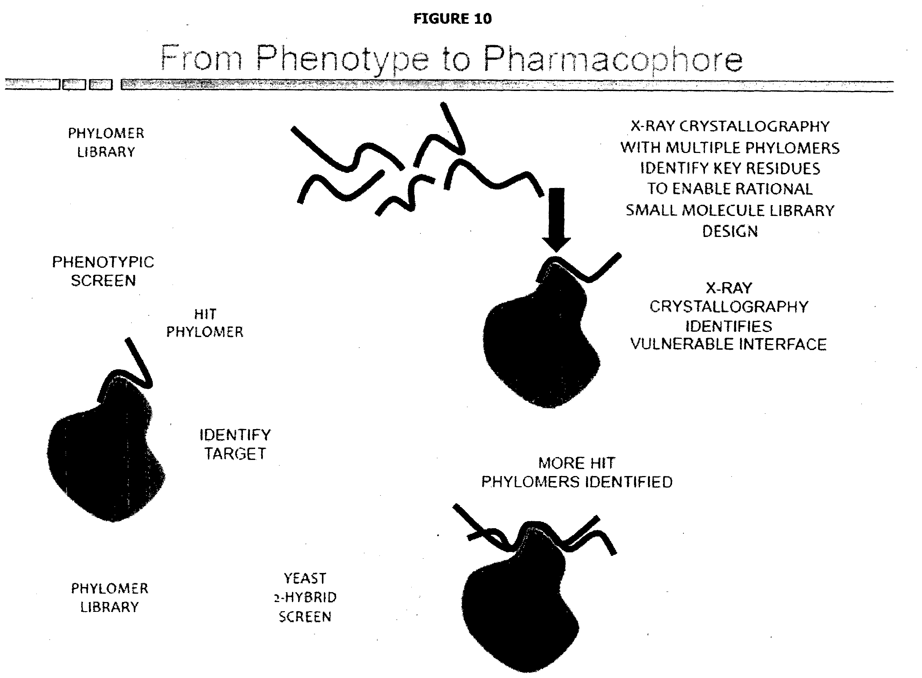

Accordingly, described herein is **a method of identifying a ligand** which binds to a target protein (in particular at the interaction site), wherein the target protein modulates the phenotype of a mammalian cell, such as a phenotype that is not death and/or reduced growth, said method comprising the step of identifying, using *in silico methods,* the structure of a ligand which is dockable (for example, can be successfully docked, or docks given the particular testing parameters) to a three dimensional structure of an interaction site of said target protein, wherein said three dimensional structure is determined by a method described herein. FIGURE 10 depicts one possible embodiment of such aspect.

Standard techniques of *in silico* drug discovery may be employed to determine the fit (or dockability) of the ligand into the interaction site. For example, a database may be virtually (ie *in-silico*) screened to identify the structure of a ligand which matches the interaction site. For example, a library of structures of commercially-available small molecules may be virtually filtered to find molecules with appropriate chemical groups, bond angles etc to occupy the interaction site defined by the Phylomer binding poses.

In some embodiments, a virtual screen of this subset of small molecules may be run against the interaction site using virtual screening methodologies such as GlideTM (Schrodinger Inc, USA). This would generate a smaller number of molecules of interest for further investigation.

Alternatively, chemical units (such as substructures, building blocks or functional groups) may be virtually assembled in a step-wise manner in the interaction site to produce a structure of a fitted ligand. For example, the structure of a ligand may be identified or assembled which contains molecular sites which interact with the molecular sites on the target protein with bond lengths and angles identified as optimal from the binding of the sub-population of Phylomers.

A ligand which fits (or is dockable) into the ligand binding space may be identified, obtained and/or synthesised. The ligand may be contacted with the target protein and binding determined. Accordingly, the method of this aspect described herein may further comprise the steps of: providing a ligand having said identified structure; contacting said ligand with said target protein; and determining (for example empirically) the binding of said ligand to said target protein.

The ligand may be tested in an assay to see if it displaces a Phylomer from the target protein. Standard displacement/binding assay platforms, such as Alpha-LISA or fluorescence polarisation, may be employed. Accordingly, the method may further comprise the steps of: providing one or more Phylomers that bind to said interaction site; and determining the ability of said ligand to compete with one or more of said Phylomers for binding to said target protein, and optionally the step of determining the ability of said ligand to modulate said phenotype of a mammalian cell capable of displaying said phenotype.

Described herein is **a method of identifying a target protein** which modulates the phenotype of a mammalian cell, such as a phenotype that is not death and/or reduced growth, said method comprising the steps:
exposing a population of *in-vitro* cultured mammalian cells capable of displaying said phenotype to a library of Phylomers;
identifying a cell in the population which displays an alteration in said phenotype following said exposure;
identifying a Phylomer that alters said phenotype of the cell;
providing the identified Phylomer; and
identifying a cellular protein which binds to said provided Phylomer,
said cellular protein being a target protein which modulates said phenotype of the mammalian cell.

Described herein is **a method of identifying a Phylomer** which modulates the phenotype of a mammalian cell, such as a phenotype that is not death and/or reduced growth, said method comprising the steps: exposing a population of *in-vitro* cultured mammalian cells capable of displaying said phenotype to a library of Phylomers; identifying a cell in the population which displays an alteration in said phenotype following said exposure; and identifying a Phylomer that alters said phenotype of the cell, said Phylomer being one which modulates said phenotype of the mammalian cell.

With a Phylomer and a target protein identified as a binding pair, such as by employing one or more method described herein, this interaction may be employed in an assay to identify a compound (such as a small molecule) that modulates the binding of the Phylomer to the target protein (in particular at the interaction site). Therefore, in one embodiment the method further comprises the steps:
providing said target protein and said provided Phylomer; and
determining the effect of a test compound on the binding of said Phylomer to said target protein (in particular at the interaction site),
wherein a test compound which modulates the degree of binding of said Phylomer to said target protein is a candidate modulator of said phenotype of the mammalian cell.

The person of ordinary skill will recognise that particular embodiments for such methods may include those described for other methods described herein that employ a similar step of feature.

Also described herein is **a method of identifying a compound** (such as small molecule) which is a candidate modulator of the phenotype of a mammalian cell, other than death and/or reduced growth, said method comprising the steps:
exposing a population of *in-vitro* cultured mammalian cells capable of displaying said phenotype to a library of Phylomers;
identifying a cell in the population which displays an alteration in said phenotype following said exposure;
identifying a Phylomer that alters said phenotype of the cell;
providing the identified Phylomer;
identifying a cellular protein which binds to said provided Phylomer, said cellular protein being a target protein which modulates said phenotype of the mammalian cell;
providing said target protein and said provided Phylomer; and
determining the effect of a test compound on the binding of said Phylomer to said target protein,
wherein a test compound which modulates the degree of binding of said Phylomer to said target protein is a candidate modulator of said phenotype of the mammalian cell.

In certain embodiments, such method further comprises the steps of contacting said candidate modulator with a mammalian cell capable of displaying said phenotype; and_determining the ability of said candidate modulator to modulate said phenotype of the mammalian cell.

The person of ordinary skill will recognise that particular embodiments for such methods may include those described for other methods described herein that employ a similar step of feature. The determination of the effect of a test compound on the binding of the Phylomer to the target protein may be conducted using standard displacement/binding assay platforms such as Alpha-LISA or fluorescence polarisation and others as described herein.

With a target protein identified as modulating the phenotype of a mammalian cell, such as by employing one or more method described herein, this target protein may be employed in an assay to characterise an interaction site on the target protein involved in the modulation of the phenotype. Accordingly, described herein is **a method of characterising an interaction site on a target protein** which modulates the phenotype of a mammalian cell, such as a phenotype that is not death and/or reduced growth, said method comprising the steps:
providing said target protein;
providing a population of Phylomers which bind to said target protein;
empirically determining the binding configuration of at least one Phylomer within said population to said target protein; and
identifying: (i) locations of binding energy; and/or (ii) the orientation of at least one side chain of said Phylomer that interacts with said protein target, in either case by analysis of said binding configuration,
thereby characterising the interaction site on said target protein.

In certain embodiments of such method, the target protein is a component of a cellular signalling pathway, and/or the target protein is identified by a method described herein. The person of ordinary skill will recognise that particular embodiments for such methods may include those described for other methods described herein that employ a similar step of feature.

Also described herein is **a peptide or protein** that comprises the amino acid sequence of a Phylomer identifiable (eg identified) by a method described herein. Such a Phylomer or amino acid sequence that is first identified by a method described herein may be designated a "base" Phylomer or sequence, and also described herein is a fragment, variant and/or derivative of such base Phylomer or base amino acid sequence.. Preferably the peptide or protein, and/or a fragment, variant or derivative thereof: (i) modulates the phenotype of a mammalian cell, such as a phenotype that is not death and/or reduced growth; and/or (ii) binds to a target protein that modulates the phenotype of a mammalian cell, such as a phenotype that is not death and/or reduced growth.

Fragments of proteins or peptides described herein may comprise a sequence of a protein or peptide as defined herein, which is, with regard to its amino acid sequence (or its encoded nucleic acid molecule), N-terminally, C-terminally and/or intrasequentially truncated compared to the amino acid sequence of the original (native) protein (or its encoded nucleic acid molecule). Such truncation may thus occur either on the amino acid level or correspondingly on the nucleic acid level. A sequence identity with respect to such a fragment as defined herein may therefore preferably refer to the entire protein or peptide as defined herein or to the entire (coding) nucleic acid molecule of such a protein or peptide. Fragments of proteins or peptides in the context of the description herein may furthermore comprise a sequence of a protein or peptide as defined herein, which has a length of about 6 to about 20 or even more amino acids, preferably having a length of about 8 to about 10 amino acids, e.g. 8, 9, or 10, (or even 6, 7, 11, or 12 amino acids), preferably having a length of about 13 or more amino acids, e.g. 13, 14, 15, 16, 17, 18, 19, 20 or even more amino acids, wherein these fragments may be selected from any part of the amino acid sequence. Fragments of proteins or peptides may comprise at least one epitope of those proteins or peptides. Furthermore also domains of a protein, like the extracellular domain, the intracellular domain or the transmembran domain and shortened or truncated versions of a protein may be understood to comprise a fragment of a protein.

Variants of proteins or peptides may be generated having an amino acid sequence which differs from the original (eg base) sequence in one or more mutation(s), such as one or more substituted, inserted and/or deleted amino acid(s). Preferably, these fragments and/or variants have the same biological function or specific activity compared to the full-length native protein, e.g. its specific antigenic property. Variants of proteins or peptides may comprise conservative amino acid substitution(s) compared to their native, i.e. non-mutated physiological, sequence. Those amino acid sequences as well as their encoding nucleotide sequences in particular fall under the term variants as defined herein. Substitutions in which amino acids, which originate from the same class, are exchanged for one another are called conservative substitutions. In particular, these are amino acids having aliphatic side chains, positively or negatively charged side chains, aromatic groups in the side chains or amino acids, the side chains of which can enter into hydrogen bridges, e.g. side chains which have a hydroxyl function. This means that e.g. an amino acid having a polar side chain is replaced by another amino acid having a likewise polar side chain, or, for example, an amino acid characterized by a hydrophobic side chain is substituted by another amino acid having a likewise hydrophobic side chain (e.g. serine (threonine) by threonine (serine) or leucine (isoleucine) by isoleucine (leucine)). Insertions and substitutions are possible, in particular, at those sequence positions which cause no modification to the three-dimensional structure or do not affect the binding region. Modifications to a three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed.), Elsevier, Amsterdam).

In order to determine the percentage to which two sequences are identical, e.g. nucleic acid sequences or amino acid sequences as defined herein, preferably the amino acid sequences encoded by a nucleic acid sequence of the polymeric carrier as defined herein or the amino acid sequences themselves, the sequences can be aligned in order to be subsequently compared to one another. Therefore, e.g. a position of a first sequence may be compared with the corresponding position of the second sequence. If a position in the first sequence is occupied by the same component as is the case at a position in the second sequence, the two sequences are identical at this position. If this is not the case, the sequences differ at this position. If insertions occur in the second sequence in comparison to the first sequence, gaps can be inserted into the first sequence to allow a further alignment. If deletions occur in the second sequence in comparison to the first sequence, gaps can be inserted into the second sequence to allow a further alignment. The percentage to which two sequences are identical is then a function of the number of identical positions divided by the total number of positions including those positions which are only occupied in one sequence. The percentage to which two sequences are identical can be determined using a mathematical algorithm. A preferred, but not limiting, example of a mathematical algorithm which can be used is the algorithm of Karlin et al. (1993), PNAS USA, 90:5873-5877 or Altschul et al. (1997), Nucleic Acids Res., 25:3389-3402. Such an algorithm is integrated in the BLAST program. Sequences which are identical to the sequences described herein to a certain extent can be identified by this program. A variant of a protein or peptide may have at least 70%, 75%, 80%, 85%, 90%, 95%, 98% or 99% amino acid identity over a stretch of 10, 20, 30, 50, 75 or 100 amino acids of such protein or peptide. Analogously, a variant of a nucleic acid sequence may have at least 70%, 75%, 80%, 85%, 90%, 95%, 98% or 99% nucleotide identity over a stretch of 10, 20, 30, 50, 75 or 100 nucleotide of such nucleic acid sequence.

A derivative is a molecule that is derived from another molecule, such as said peptide or protein. A derivative of a peptide or protein also encompasses fusions comprising a peptide or protein used as described herein. For example, the fusion comprises a label, such as, for example, an epitope, e.g., a FLAG epitope or a V5 epitope or an HA epitope. For example, the epitope is a FLAG epitope. Such a tag is useful for, for example, purifying the fusion protein. The term "derivative" of a peptide or protein also encompasses a derivatised peptide or protein, such as, for example, a peptide or protein modified to contain one or more-chemical moieties other than an amino acid. The chemical moiety may be linked covalently to the peptide or protein e.g., via an amino terminal amino acid residue, a carboxyl terminal amino acid residue, or at an internal amino acid residue. Such modifications include the addition of a protective or capping group on a reactive moiety in the peptide or protein, addition of a detectable label, and other changes that do not adversely destroy the activity of the peptide or protein compound. For example, a derivative may comprise a PEG moiety, radionuclide, coloured latex, etc. A derivative generally possesses or exhibits an improved characteristic relative to a e.g., enhanced protease resistance and/or longer half-life and/or enhanced transportability between cells or tissues of the human or animal body and/or reduced adverse effect(s) and/or enhanced affinity or immunogenicity. WO 2010/003193 describes various methodologies to provide peptide or protein derivatives which may be employed separately or in combination using standard procedures known to the person of ordinary skill, including derivatisation of a protein or peptide by e.g. PEGylation, HESylation, PASylation, or glycosylation.

In a particular embodiment, a peptide or protein described herein comprises the amino acid sequence of a (base) peptide selected from the list consisting or: 4G9, 6F6, 6G8, 10B11, 25C3, 44B2 and 48E6. Also described herein is a fragment, variant and/or derivative of a peptide or protein comprising such amino acid sequences. In particular such embodiments, such moiety binds to MAP4K4.

In certain embodiments, a peptide or protein, or a fragment, variant and/or derivative thereof, as described herein, is in isolated or purified form. A sample is considered purified if less than about 50%, 40%, 20%, 10%, 5%, 2.5% or 1% of the amount (eg my mass) of the sample contains undesired or undefined components, such as impurities.

Also described herein is **a use of the peptide or protein** as described above, or a fragment, variant and/or derivative thereof, to: (i) modulate a phenotype of a mammalian cell, such as a phenotype that is not death and/or reduced growth; and/or (ii) to bind to a target protein that modulates a the phenotype of a mammalian cell, such as a phenotype that is not death and/or reduced growth. Such use may be employed in eg *in-vitro* or *ex-vitro* methods such as a method as described here. Alternatively, such use may be employed *in-vivo,* such as in pharmacological uses to modulate the activity of a target protein to alter a phenotype of a cell in the body, for example to seek to achieve a pharmacologically relevant effect. Also described herein is **a nucleic acid** comprising a sequence capable of encoding a peptide or protein described herein (or a fragment, variant and/or derivative thereof). Preferably, a nucleic acid described herein comprises a nucleotide sequence selected from those in TABLE 2. In certain embodiments, a nucleic acid described herein is in isolated or purified form, and/or one that is a recombinant nucleic acid. A nucleic acid described herein may be employed to produce, such as to manufacture, a peptide or protein, such as one described herein.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment described herein and apply equally to all aspects and embodiments which are described.

Certain aspects and embodiments described herein will now be illustrated by way of example and with reference to the description, figures and tables set out herein. Such examples of the methods, uses and other aspects as described herein are representative only, and should not be taken to limit the scope of the present invention to only such representative examples.

### EXAMPLES

### Phylomer library construction

### Creation of Phylomer fragments

Genomic DNA from sequenced bacterial genomes (TABLE 1) was obtained, for example from the American Type Tissue Culture, and used as template for random low-temperature linear amplification. This was carried out using Klenow fragment by primer extension using random degenerate primers (BGF-N6 and BGF-N9) containing a FLAG tag in an amplification protocol designed so that the oligonucleotides would anneal with equal efficiency to either AT or GC rich sequences in order to minimize amplification bias. Amplification was carried out over four rounds as follows:
- Amplification Round 1: 3.33uM Primer BGF-N6, IX Klenow buffer, 200 uM dNTPs, Klenow fragment of DNA polymerase I, PEG (8500) in total volume of 30ul. Mix primer, DNA, and water; boil for 3-5 min, snap cool on ice and then transfer to tube containing the other reagents. Incubate 15 °C for 30 min, room temperature for 2 hours, then 37 °C for 15 min.
- Amplification Round 2: Boil tube 5 min, snap cool, add 0.5ul Klenow enzyme. Incubate 15 °C for 30 min, room temperature for 2 hours, then 37 °C for 15 min.
- Amplification Round 3: Boil tube 5 min, snap cool, add: 4µl BGF-N9 primer (25uM), 1µl 10X buffer, 3 ul dNTPs (2mM), 0.5 ul Klenow, 1.5 ul water. Incubate 15 °C for 30min, room temperature for 2 hours, 37 °C for 15 min.
- Amplification Round 4: Boil tube 5 min, snap cool, add 0.5µl Klenow enzyme. Incubate 15 °C for 30 min, room temperature for 2 hours, then 37 °C for 15 min. Products were purified using Amplicon spin columns.

Primer sequences used are as follows, 5' to 3', restriction sites shown in lower case:
- BGF-N6: GACTACAAGGACGACGACGACAAGGCTTATCAATCAATCANNNNNN (SEQ ID NO: 1)
- BGF-N9: GACTACAAGGACGACGACGACAAGGCTTATCAATCAATCANNNNNNNNN (SEQ ID NO: 2)
- BGF-F5: GAGAGgaattcAGGTCAGACTACAAGGACGACGACGACAAG (SEQ ID NO: 3)
- BGF-R6-Acc651: GAGAGggtaccAGGTCAGACTACAAGGACGACGACGACAAG (SEQ ID NO: 4)

Products from these amplifications were used as template for conventional PCR to both amplify the genomic fragments and to add restriction enzyme digestion sequences at the 5' ends of all amplified fragments using primers BGF-F5 and BGF-R6. After 25 PCR cycles, the amplified products were assessed using gel electrophoresis to confirm the presence of a smear of fragments ranging in size from 50 to 1000 bp. Products were purified using Amplicon spin columns.

**TABLE 1: Genomes used to construct Phylomer libraries used in these examples**

| **List 1** | **List 2** |
|---|---|
| *Aeropyrum pernix* | *Aquifex aeolicus* |
| *Archeaoglobus fulgidis* | *Bacillus subtilis* |
| *Bacillus subtilis* | *Bordetella pertussis* |
| *Bordetella pertussis* | *Borrelia burgdorferi* |
| *Borrelia burgdorferi* | *Chlamydia trachomatis* |
| *Campylobacter jejuni* subsp. *jejuni* | *Escherichia coli K12* |
| *Chlorobium tepidum* | *Haemophilus influenzae* |
| *Clostridium acetobutylicum* | *Helicobacter pylori* |
| *Deinococcus radiodurans* | *Methanobacterium thermoautotrophica* |
| *Escherichia coli K12* | *Methanococcus jannashii* |
| *Haemophilus influenzae* | *Neisseria meningitides* |
| *Halobacterium salinarum* | *Pyrococcus horikoshi* |
| *Helicobacter pylori* | *Pseudomonas aeruginosa* |
| *Listeria innocua* | *Synechocystis PCC 6803* |
| *Methanococcus jannaschii* | *Thermoplasma volcanicum* |
| *Neisseria menigitidis* | |
| *Pseudomonas aeruginosa* | |
| *Pyrococcus horikoshii* | |
| *Salmonella enterica* subsp. *enterica* serovar. *Thyphimurium* | |
| *Shigella flexneri* | |
| *Staphylococcus aureus* | |
| *Streptomyces avermitilis* | |
| *Sulfolobus solfataricus* | |
| *Thermoplasma volcanicum* | |
| *Thermotoga maritime* | |

| | |
|---|---|
| *Construction of Phylomer Libraries* | |

To generate a phylomer library for target-based screening (in these examples using yeast 2-hybrid based screening), the biodiverse gene fragments obtained as above from the species in List 1 (TABLE 2) were cloned into the pCR8/GW/TOPO-TA entry vector of the Gateway recombination cloning system (Invitrogen) to make a library of 1.7 x 10⁷cfus. The inserts of this entire library were transferred using Gateway recombination cloning technology into a 'destination' yeast two- hybrid prey vector derived from pJG4-5 that was compatible with the membrane-based Ras-Recruitment two-hybrid system (Hennemann et al., 2003; Walhout et al., 2000).

To generate the mammalian expression Phylomer library for phenotype screens, the primary Phylomer entry library in the pCR8/GW/TOPO-TA Gateway entry vector was transferred into a mammalian expression destination vector pcDNA3.1/nV5-DEST using Gateway recombination cloning technology.

To investigate the efficiency of phenotypic screening using Phylomer libraries, in this case a sub-library of 3120 individual clones was created by randomly selecting clones from the above mammalian expression Phylomer library, growing 2ml cultures of such selected individual clones and recovering mini-prep DNA using the PureLink 96 Plasmid Purification System (Invitrogen). A control vector was also generated by Gateway recombination of the GusB coding sequence from the pENTR-GusB vector into pcDNA3.1/nV5-DEST. Plasmid DNA from 6 different wells on each plate was quantitated by spectrophotometry to generate an average DNA concentration.

### Phenotypic screening of Phylomer libraries

We were surprised to find a very high hit rate following phenotypic screening of mammalian cells with Phylomer libraries. From a limited library of about 3,000 clones, 14 initial hits were selected: a hit rate far higher than previously reported for random peptide libraries (Park and Raines, 2000; Nat Biotechnol 18: 847-851; Xu and Luo 2002; Xu et al., 2001). Confirmatory experiments showed that Phylomers so identified exhibited specificity for the tested phenotype; validating this approach to identify diverse Phylomer peptides that have phenotypic effects on mammalian cells.

The mini-library of 3120 individual transfection-ready plasmids was used in a microtitre plate-based transfection assay system (ie, and array-based format) in the U2oS cell line, and the effects of the Phyomers expressed from this mini-library were assessed upon PMA-mediated API-driven luciferase reporter expression. The previously identified Phylomer PYC36 (GLQGRRRQGYQSIKP SEQ ID NO: 5) - found by reverse target-based yeast 2-hybrid screening against c-Jun bait, and known to affect API-dependent transcription (Mead et al., 2010 J. Neurochem, 112: 258-270) - was used as positive control.

Briefly, in triplicate plates, 1x10⁵ U2oS cells were plated overnight and co-transfected with 100ng of both AP1-luciferase reporter (Stratagene) and either an individual Phylomer construct from the mini-libary, or positive control (pcDNA3.1/PYC36) or vector control (pcDNA3.1/nV5-DEST-GusB). PMA (100nM) was used to induce AP1-dependent luciferase expression 6 hours post-transfection, and luciferase activity was measured 24 hours later (SteadlyGlo, Promega).

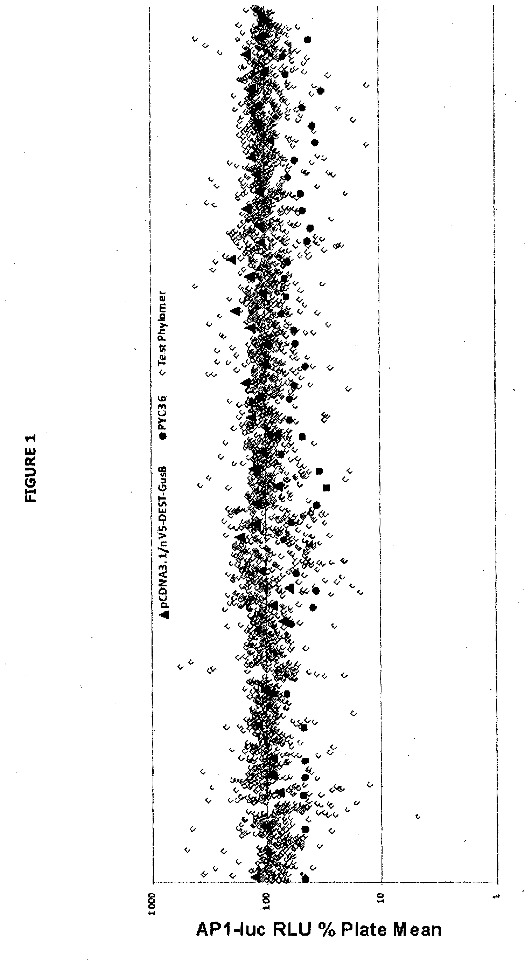

Whilst the majority of Phylomer clones showed minimal effects upon API-dependent luciferase activity, we were surprised to observe a relatively high number of clones that, like PYC36, inhibited transcription, and also a number which apparently enhanced reporter activity (FIGURE 1).

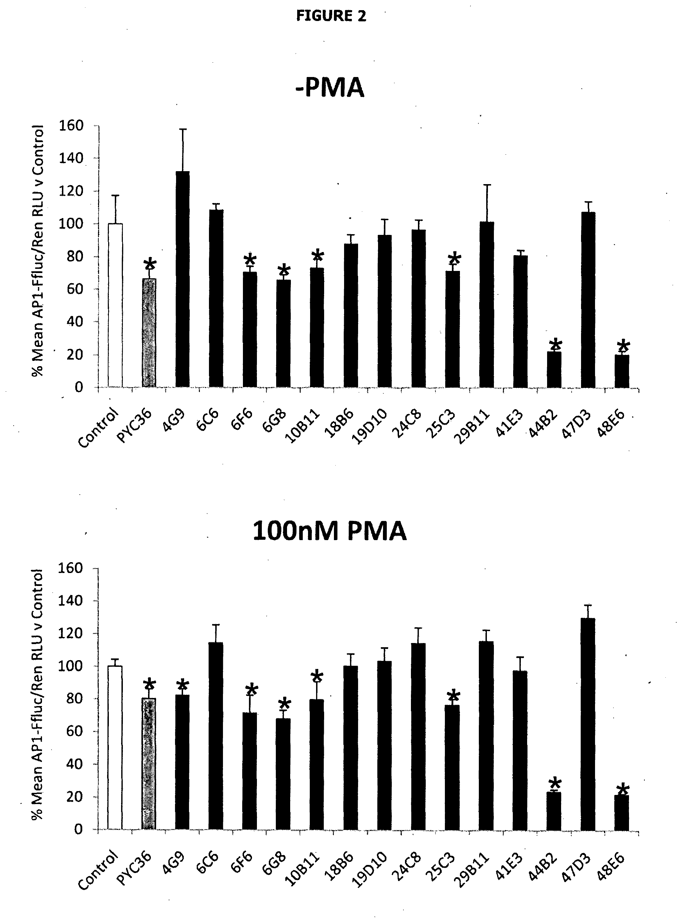

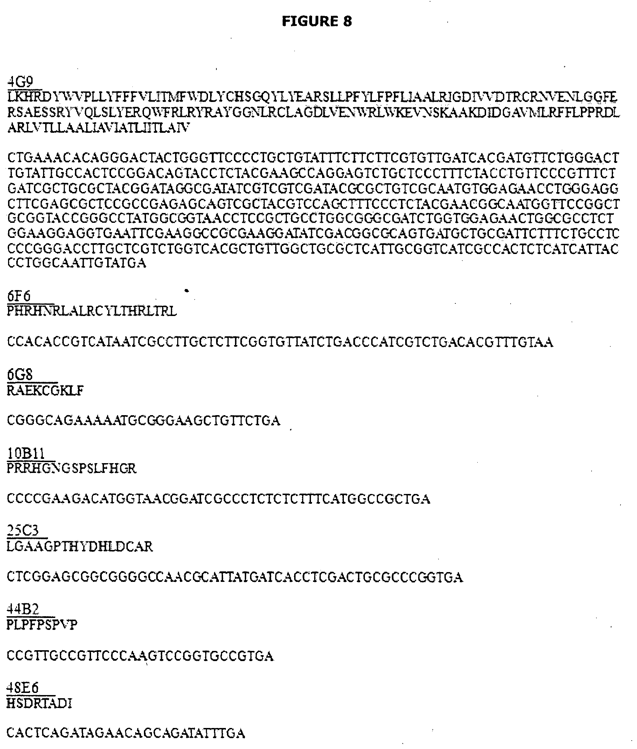

From this primary screen, we concentrated on the PYC36-like, API-inhibitory Phylomers, and selected 14 initial hits. Of these, 7 (TABLE 2 and FIGURE 8) were confirmed to inhibit AP1-luciferase activity in a secondary reporter assay normalised to Renilla luciferase expression (FIGURE 2). Briefly, Phylomer DNA of the primary hits was re-prepared from the original clones selected for the mini-library (Endo-Free Maxi-Prep, Qiagen), and 5x10⁵ U2oS cells were co-transfected with 800ng DNA comprising AP1-luciferase or Srxn1-luciferase (Papadia et al., 2008) (Firefly), pRL-TK (Renilla) and Phylomer plasmid. Reporter activity/inhibition was calculated by normalizing Firefly activity to Renilla.

**TABLE 2: Amino acid sequences of API-inhibitory Phylomers identified by phenotypic screening, and corresponding nucleic acid sequences encoding same**

| **Phylomer** | **Amino acid sequence** | **Nucleic acid sequence** |
|---|---|---|
| **4G9** | | |
| **6F6** | PHRHNRLALR CYLTHRLTRL (SEQ ID NO: 8) | |
| **6G8** | RAEKCGKLF (SEQ ID NO: 10) | CGGGCAGAAA AATGCGGGAA GCTGTTCTGA (SEQ ID NO: 11) |
| **10B11** | PRRHGNGSPS LFHGR (SEQ ID NO: 12) | |
| **25C3** | LGAAGPTHYD HLDCAR (SEQ ID NO: 14) | |
| **44B2** | PLPFPSPVP (SEQ ID NO: 16) | CCGTTGCCGT TCCCAAGTCC GGTGCCGTGA (SEQ ID NO: 17) |
| **48E6** | HSDRTADI (SEQ ID NO: 18) | CACTCAGATA GAACAGCAGA TATTTGA (SEQ ID NO: 19) |

Transfection of these Phylomers was not generally cytotoxic as assessed by cell viability using the Cell-Titre Blue system (Promega) (data not shown). To initially assess their specificity for API inhibition, we tested these hits against two heterologous reporter constructs designed to detect the activation of two distinct transcription factors, namely Notch and FOXO class Forkhead. Of the 7 Phylomers tested, 6 showed no effect on these reporters, while one Phylomer (4G9) generally inhibited all reporter constructs, including both Firefly and Renilla luciferases (data not shown).

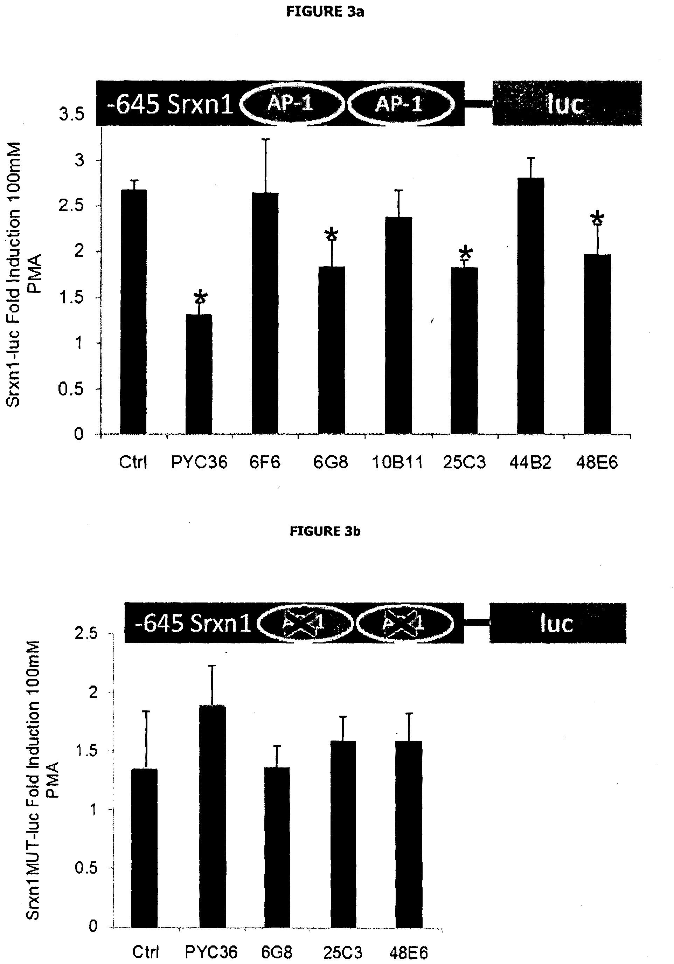

As an additional confirmatory experiment, we asked whether these 6 Phylomers specifically regulated expression of a known API target gene by testing their effect on the Sulfiredoxin (Srxn1) promoter, recently established as an API-dependent component of the anti-oxidant response mechanism (Papadia et al., 2008). Of the 6 Phylomers tested, 3 inhibited PMA-dependent activation of this promoter: 6G8, 25C3 and 48E6 (FIGURE 3a). Importantly, these Phylomers had no effect upon PMA-induced transcription from a control Srxn1 promoter in which the API response elements had been ablated by mutation (FIGURE 3b), confirming that their effect on the Srxn1 promoter is API-specific.

Collectively, these experiments suggest that Phylomers exhibiting specificity for API-dependent transcription can be recovered at high hit rates from direct phenotypic screening in cultured mammalian (human) cells using a library of nucleic acids encoding Phylomers.

### Production of identified Phylomers

The peptide 25C3 was expressed as a His-MBP tagged fusion construct from the pDEST-HisMBP expression vector in Rosetta2(DE3) cells (Nallamsetty et al., 2005). Briefly, cells were grown at 37 °C in 500ml 2YT broth (supplemented with 0.4% glucose, 50 ug/mL carbenicillin, 30 ug/mL chloramphenicol) until OD595 was 0.6, when protein expression was induced for 2 hours with ImM IPTG at 37 °C. Cells were washed in PBS (pH 8.0), lysed, by sonication (2 x 1.0 minute at 80% duty cycle) in 50mL of lysis buffer (PBS pH 8.0, 1.0 mM PMSF, Complete Protease Inhibitor Tablet), and lysates clarified by centrifugation at 43,146 g for 20 minutes. SDS-PAGE (12%) analysis confirmed that the peptide fusion was retained in the soluble fraction.

The 25C3 fusion peptide was purified in a two-step protocol on the AKTAxpress FPLC (GE Healthcare Life Sciences). Briefly, proteins were purified by affinity chromatography over MBP-Trap columns (5ml), washed with PBS (pH 8.0), eluted using a gradient of PBS pH8.0/Maltose 10 mM, then further purified by gel filtration over a HiPrep 16/60 Sephacryl S-100 column equilibrated in PBS (pH 8.0). Native-PAGE (8.0%) analysis confirmed the presence of monomeric protein species. The amount of the resulting fusion peptide was quantified using the BCA Assay kit (Pierce).

Any synthetic peptides used in this study were synthesised by Mimotopes Pty Ltd (Melbourne) to >70% purity (in vitro studies) or >95% purity (in vivo studies).

### Identification of targets that modulate the phenotype of mammalian cells

We selected Phylomer 25C3 (derived from the extremophile *Deinococcus radiodurans*) for further studies to identify its cellular binding partner. We were surprised to find that not only was a high hit rate observed from direct phenotypic screening, but that primary hits had a high stability and affinity, sufficient for direct use to identify protein targets that modulate the phenotype of mammalian cells.

Briefly, U2oS cells were transfected with a V5-tagged 25C3 expression vector (pCDNA3.1 V5-His A from Invitrogen), and cell lysates were immunoprecipitated with an anti-V5 antibody.

All LC-MS/MS experiments were performed using an Eksigent NanoLC-1D Plus (Eksigent Technologies, Dublin, CA) HPLC system and an LTQ Orbitrap mass spectrometer (ThermoFisher, Waltham, MA). Immunprecipitated proteins were protease (eg trypsin) digested using standard procedures. Separation of peptides was performed by reverse-phase chromatography using at a flow rate of 300 nL/min and an LC-Packings (Dionex, Sunnyvale, CA) PepMap 100 column. Peptides were loaded from the autosampler with 0.1% formic acid for 5 minutes at a flow rate of 10 uL/min. After this period, the valve was switched to allow elution of peptides from the precolumn onto the analytical column. Solvent A was water + 0.1% formic acid and solvent B was acetonitrile + 0.1% formic acid. The gradient employed was 5-50% B in 50 minutes. The LC eluant was sprayed into the mass spectrometer by means of a New Objective nanospray source. All m/z values of eluting ions were measured in the Orbitrap mass analyzer, set at a resolution of 7500 LTQ linear ion trap by collision-induced dissociation (CID) and MS/MS spectra were acquired. Post-run, the data was processed using Bioworks Browser (version 3.3.1 SP1, ThermoFisher). Briefly, all ms/ms data were converted to .dta (text) files using the Sequest Batch Search tool (within Bioworks). The dta files were converted to a single mgf file using a SSH script in the SSH Secure Shell Client program (Version 3.2.9 Build 283, SSH Communications Corp.). These combined files were then submitted to the Mascot search algorithm (Matrix Science, London UK) and searched against the NCBI human database, using a fixed modification of carbamidomethyl and a variable modification of oxidation (M).

Such proteomic mass spectrometry analysis identified two of the immunoprecipitating partners of the Phylomer 25C3 as MAP4K4 and Citron kinase. We noted with interest that both these proteins contain a Citron homology domain (CNH), a putative protein:protein interaction module. Importantly, MAP4K4 (also known as Nck-Interacting Kinase (NIK)) is an established upstream regulator of JNK activity (Taira et al., 2004), consistent with the identification of 25C3 as an API inhibitor in our screen. Indeed, siRNA-mediated knockdown of MAP4K4 in U2oS cells inhibited AP1-dependet luciferase expression, as did 25C3 over-expression (data not shown).

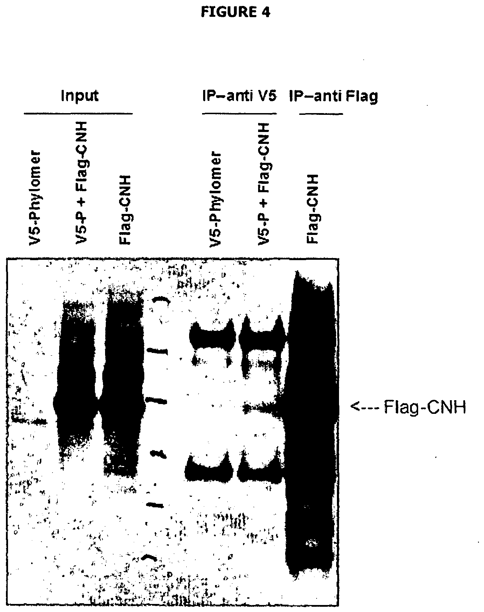

We next confirmed the interaction between 25C3 and MAP4K4 ex vivo by immunoprecipitating a FLAG-tagged MAP4K4 CNH domain using V5-tagged 25C3 in HEK293 cells (FIGURE 4). Briefly, HEK293T cells were transfected with V5-tagged 25C3 alone or with a pcDNA3.1 construct expressing the CNH domain of human MAP4K4 (amino acids 1002 to 1300) fused with an N-terminal Flag tag. Cell lysates were prepared in NP-40 buffer and immunoprecipitated with mouse anti-V5 antibody (Invitrogen), and then immunoblotted with mouse anti-Flag antibody (M2, Sigma-Aldrich). As a control, cells were transfected with Flag-CNH alone before being immunoprecipitated and immunoblotted with anti-Flag.

### Affinity characterisation of target-Phylomer binding

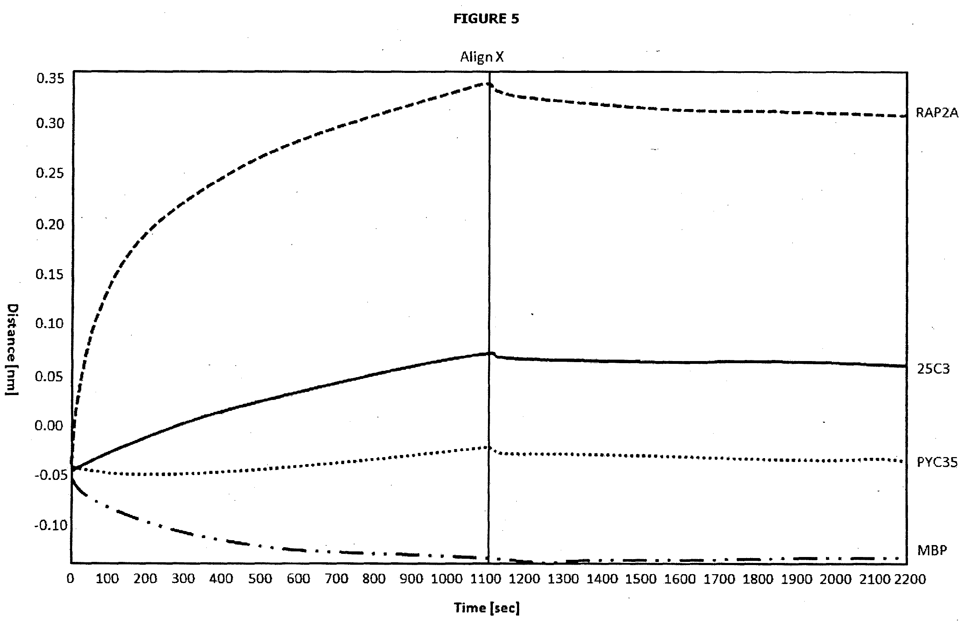

The binding affinity of the Phylomer-target interaction was characterised by measuring the in vitro binding affinity of 25C3 and the CNH domain of MAP4K4 using Bio-Layer Interferometry (FIGURE 5 and TABLE 3).

**TABLE 3: Characterisation of binding affinity of API-inhibitory Phylomer 25C3 to MAP4K4-CNH compared to controls**

| **Ligand (1µM)** | **K-on (1/Ms)** | **K-dis (1/s)** | **KD (M)** | **Full R2** |
|---|---|---|---|---|
| MBP-RAP2A | 5.56 x 10-3 | 5.32 x 10-5 | 9.58 x 10-9 | 0.93 |
| MBP-25C3 | 1.87 x 10-3 | 4.40 x 10-5 | 2.35 x 10-8 | 0.98 |
| MBP-PYC35 | 4.23 x 10-1 | 7.27 x 10-6 | 7.27 x 10-6 | 0.88 |
| MBP | 1.00 x 10-4 | 2.65 x 10-7 | 2.65 x 10-11 | -13.50 |

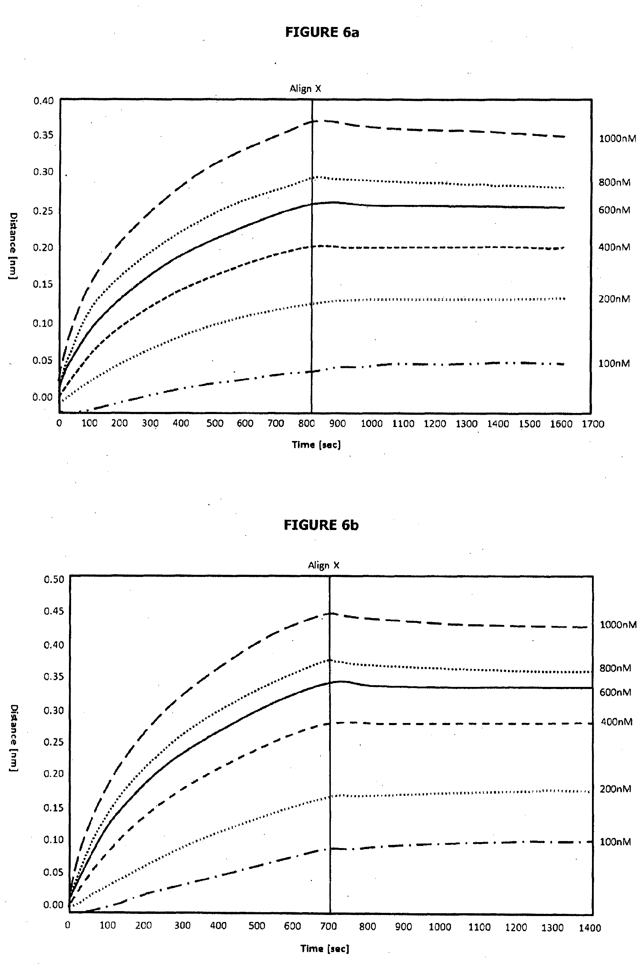

Analysis of the binding kinetics over a dose-titration of the ligand shows the surprising finding that the 25C3 Phylomer binds to MAP4K4 with a Kd of 28nM, only 5-fold lower binding affinity than to a natural ligand, RAP2A (Machida et al., 2004) measured as 4.8nM in the same experimental system as a positive control (FIGURE 6 and TABLE 4).

**TABLE 4: Binding affinity of API-inhibitory Phylomer 25C3 to MAP4K4-CNH compared to RAP2A**

| | **MBP-25C3** | **MBP-RAP2A** |
|---|---|---|
| **KD (M)** | 2.80 x 10-8 | 4.77 x 10-9 |
| **K-on (1/Ms)** | 3.50 x 10-3 | 2.74 x 10-3 |
| **K-dis (1/s)** | 1.00 x 10-4 | 1.30 x 10-5 |
| **R-squared** | 0.97 | 0.99 |

Relative binding characteristics of RAP2A, 25C3 and PYC35 (a negative control peptide having the sequence AYQSIRSGGIESSSKRER SEQ ID NO: 20; Mead et al, 2010) each as a MBP fusion, to MAP4K4-CNH were measured using Octet-RED (ForteBio). Data were acquired with the Data Acquisition software version 6.2 and all steps/dilutions were performed in baseline buffer (PBS pH 8.0) at a rotation rate of 1000 rpm. Briefly, biotinylated MAP4K4-CNH (45 ug/mL) was immobilised onto pre-equilibrated streptavidin-coated biosensors. Following baseline equilibration, the MAP4K4-CNH-coated sensors were exposed to RAP2A, PYC35 and 25C3 (at 1.0 mM) followed by dissociation in baseline buffer for 2000 seconds at each step. The binding affinities of RAP2A and 25C3 to MAP4K4-CNH were measured by exposing immobilised biotinylated MAP4K4-CNH to RAP2A and 25C3 over a concentration range of 100-1000 nM as described.

Binding kinetic data were processed using the Savitzky-Golay filter prior to analysis and evaluated with ForteBio Data Analysis software version 6.3. To determine the relative binding kinetics of RAP2A, 25C3 and PYC35 to MAP4K4-CNH, lines of best fit were generated locally based on a 1:1 model. To determine the binding affinities of RAP2A and 25C3 to MAP4K4-CNH the lines of best fit were generated globally based on a 1:1 model to derive kₒₙ, k_{off}, and K_{D} values.

### Assay to identify modulators of target-Phylomer binding (prophetic example)

The Bio-Layer Interferometry assay described above is used to identify compounds that affect the degree of binding of a Phylomer to its target protein. Briefly, the a 25C3/MAP4K4-CNH binding assay is established as above, but with the further inclusion of a molar excess (for example10mM) of test compound, and the effect of the test compound on the K_{D} value is determined. Compounds that are able to compete with, disrupt and/or inhibit the binding of 25C3 to MAP4K4-CNH are considered compounds that bind to the target protein.

A compound identified by such assay, is then tested for its ability to modulate the desired phenotype of mammalian cells, such as by contacting of the compound to cells in a cell-migratory assay, such as one described below.

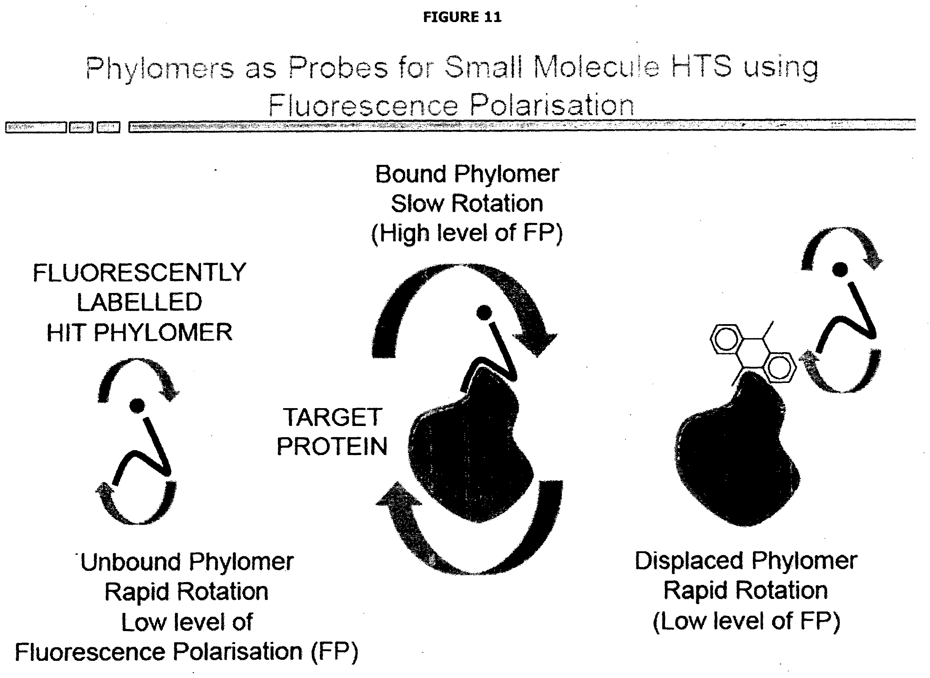

In an alternative approach, fluorescence polarisation - a homogenous assay suited to the analysis of binding between two molecules of significantly different molecular weight - is used for the identification of small molecules that can interfere with the Phylomer-target protein interaction (FIGURE 11).

The small binding partner, in this case a Phylomer peptide (for example 25C3) that binds to a target protein (for example MAP4K4), is labelled with a fluorophore (for example TAMRA or Alexafluor-488) and exposed to linearly polarised light. When the excited small binding partner is bound to the large binding partner, in this case the target protein, the resulting fluorophore-labelled complex has a high molecular weight, resulting in slow tumbling and thus a relatively uniform spatial orientation of the fluorophore at the time of fluorescence emission that is detected as a high degree of fluorescence polarisation.

In contrast, if the excited small binding partner is unbound (for example in the presence of an inhibitor of the interaction), they will rotate more rapidly, resulting in a more heterogeneous spatial orientation at the time of fluorescence emission that is detected as a low degree of fluorescence polarisation.

Binding experiments are measured using a suitable plate reader, for example the PHERAstar Plus plate reader (BMG Labtech) or the Paradigm plate reader (Beckman Coulter) and using black multiwell plates with a non-binding surface. Upon excitation with linearly polarized light of a suitable wavelength to excite the fluorophore, the fluorescence intensities parallel and perpendicular to the plane of the original excitation are recorded as fluorescence polarisation values.

Fluorescence polarization values can be multiplied by 1000 and expressed in mP. For competition assays using libraries of small molecules percentage inhibition is calculated and the data is plotted as % inhibition. From this data the half maximal inhibitory concentration (IC50) is determined for a small molecule inhibitor of the interaction.

Small molecule inhibitors identified from either of these assays are then tested in a phenotypic screen (for example the AP-1 dependant luciferase assay) to assess if they can replicate the phenotype of the original hit Phylomer. Alternatively, complementary phenotypic screens may be used to investigate the ability of such small molecules to modulate a desired phenotype of mammalian cells, such as the assay described below.

### Phenotypic modulation by binding Phylomers

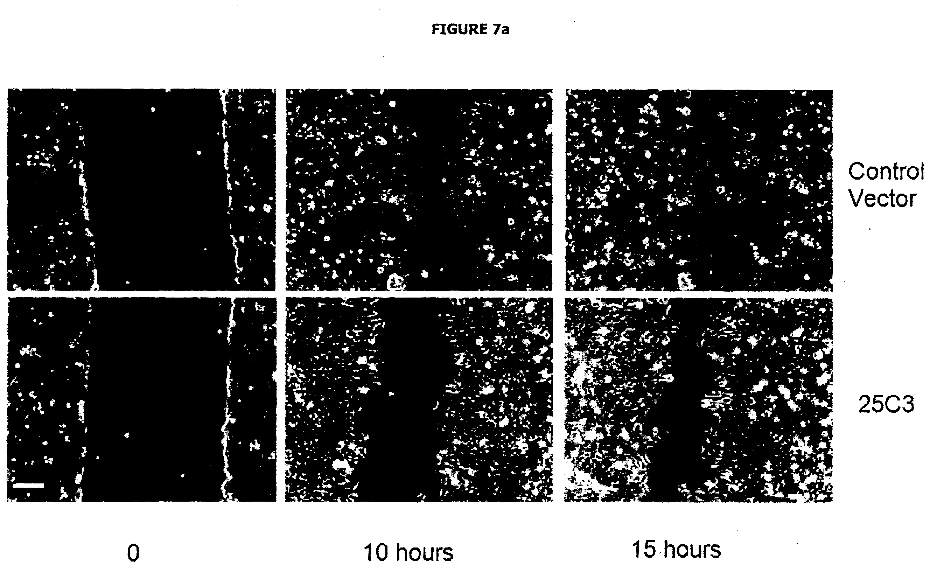

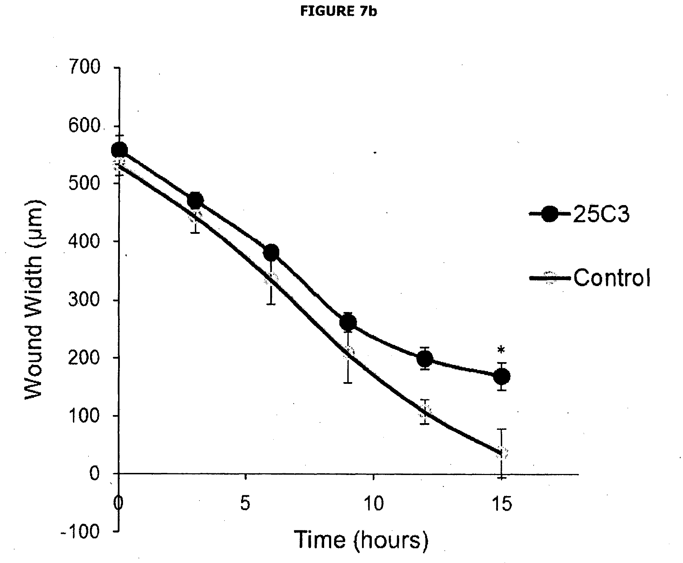

We sought to demonstrate that 25C3 over-expression could deflect a MAP4K4-dependent cellular response. Recent data has shown that MAP4K4 is a pro-migratory kinase, regulating cellular movement during embryonic development (Xue et al., 2001) and in a model cell migration assay (Collins et al., 2006). We therefore over-expressed 25C3 in U2oS cells and used time- lapse photography to investigate the time taken for cells to close the defect (FIGURE 7). 25C3 significantly retarded cellular migration and scratch closure, such that the defect remained open 24 hours after scratching, demonstrating that 25C3 can phenocopy the effects of MAP4K4 inhibition.

Briefly, U2oS cells were seeded at ∼ 0.2x10⁶ cells per chamber of 2-sample chamber slides (Nunc Lab-Tek 177429) in 1ml of antibiotic free DMEM (Invitrogen) supplemented with 10% fetal calf serum. 24 hours after plating 1.6 ug of plasmid DNA was transfected using Lipofectamine2000 according to the manufacturer's instructions. 24h after transfection, a scratch wound was created in a confluent monolayer of U2oS cells with a sterile 10 ul tip, cells were washed twice with PBS, and the medium replaced with Leibovitz's L-15 Medium (Invitrogen 21083-027) supplemented with 10% fetal calf serum. Slides were mounted on a Zeiss time-lapse microscope rig with inbuilt temperature and CO2 control. Images of the wound area were taken every 5 min for 24h. Images were analysed and the width of the wound was measured using Velocity software (Perkin Elmer).

### Target-based screening to augment the provision a population of binding Phylomers (prophetic example)

We use a novel cytoplasmic screening system analogous to the SOS-Recruitment- System (Aronheim et al., 1997) based on the activation of a mitogenic signaling pathway in the yeast *Saccharomyces cerevisiae.* In brief, the recombinant bait protein fuses human MAP4K4-CNH to the coding region of truncated hSos1. An expression plasmid, allowing constitutive expression of the bait is co-transformed (Gietz and Schiestl, 2007) with a Phylomer peptide library (prepared as above) into a cdc25-2 yeast strain. Phylomer peptides are expressed from an inducible GAL1 promoter as fusions to a lipidation signal for membrane attachment. Interactions of bait and prey proteins are tested in a yeast strain, whose endogenous RAS pathway can be regulated by a temperature sensitive mutation (cdc 25-2). Putative interactors are shifted to the restrictive temperature (37 °C) and tested for galactose dependency to identify the MAP4K4-interacting Phylomers. The peptide coding inserts of these clones are isolated and subjected to sequencing to identify, and hence augment and provide a population of Phylomers that bind to the target protein MAP4K4.

### Empirical determination of binding configuration of target-Phylomer interactions (prophetic example)

Phylomers within a population that bind to the target protein MAP4K4 are provided by synthesis (as described above) of a plurality of Phylomers identified as above. A structurally- or sequence-diverse set of such Phylomers is selected. The target protein MAP4K4 is provided as a purified recombinant product, and for one or more of the binding Phylomers, an individual such Phylomer is either co-crystallised with purified MAP4K4 using standard procedures, or alternatively soaked into pre-established MAP4K4 crystal. The binding configurations of such Phylomers to MAP4K4 are then empirically determined by conducting X-ray diffraction crystallographic analysis on the Phylomer-liganded crystals. Experimental diffraction data are collected at an appropriate facility such as Swiss Light Source, Diamond Light Source, or European Synchrotron Radiation Facility, and such empirical data are processed with an appropriate program package such as XDS. Structures are solved by molecular replacement using Molrep5 or Phaser6 from the CCP4 program suite. Models are manually rebuilt with Coot and structures are refined using Refmac. Phylomer/target complexes are then user-assessed within a visualisation environment such as that from Schrodinger or Accelrys.

### Characterisation of interaction site (prophetic example)

One or more of binding configurations for the binding Phylomer-target protein interactions are collected as described above. Computer- and analytically-aided inspection of the binding configurations enables identification of consensus binding interactions between specific residues or positions on the Phylomer(s) and amino-acid residues of the interaction site of MAP4K4. The location of such "hot spots" of binding interaction (energy) provides one approach for the characterisation of the interaction site. Such hotspots will be obvious to one skilled in the art. For example, placement of a hydrophic Phylomer amino acid side chain into a hydrophobic pocket on the target protein surface, or placement of a charged Phylomer amino acid side chain in proximity to a charge of opposite polarity within the target protein.

Further characterisation is conducted by inspection or determination of the three-dimensional structure of the interaction site, and/or location of limited interaction binding energy.

### Identification of a ligand which binds to a target protein (hypothetical example)

Given the characterisation of the interaction site as provided by the methods described herein, we can identify, by in-silico methods, the structure of a ligand that has similar regions of binding to one or more Phylomers, or that is dockable within a computer model of the characterised interaction site. Suitable computer modelling, visualisation, virtual screening and/or docking programs including those within the Schrodinger environment (Schridinger Inc, USA), especially "Glide", and analogous programs within the Accelrys environment. Using established force-fields utilised by these environments, the relative enthalpic and entropic contributions to binding energy are predicted for a proposed small molecule ligand, and summated to provide a computed free energy of binding. In conjunction with a powerful computing resource, a virtual library of commercially available small molecule structures can be assembled and screened against the target protein, with data emerging in the form of ranked predicted free energies of binding. In this way, a virtual library consisting of several million compounds can be triaged into a much smaller library of molecules which can be more feasibly confirmed for binding to the target protein by experimental assays.

The ligand (such as a small molecule) represented by the identified structure is synthesised by routine chemical methods and provided within a suitable assay or assays. For example, the effect of the ligand so identified and provided may be tested for its ability to modulate the degree of binding between the Phylomer and target protein interaction as identified or characterised by one of the methods above. The ligand may be tested for its ability to modulate the desired phenotype of a mammalian cell, for example, in the cell-migration assay described above.

## Claims

1. A method of characterising an interaction site on a target protein as dockable by a small molecule ligand, wherein the target protein modulates a phenotype of a mammalian cell, said method comprising the steps:
exposing a population of *in-vitro* cultured mammalian cells capable of displaying said phenotype to a library of peptides of about 8 to about 180 amino acids encoded by nucleic acid fragments obtainable from genome(s) of a microorganisms and/or a eukaryotic species having a compact genome;
identifying a cell in the population which displays an alteration in said phenotype following said exposure;
identifying a peptide from said library of peptides that alters said phenotype of the cell;
providing the identified peptide;
identifying a cellular protein which binds to said provided identified peptide, said cellular protein being a target protein which modulates said phenotype of the mammalian cell;
providing said target protein;
providing a population of peptides of about 8 to about 180 amino acids encoded by nucleic acid fragments obtainable from genome(s) of a microorganisms and/or a eukaryotic species having a compact genome, wherein the peptides in said population bind to said target protein at the interaction site, wherein, prior to its provision, said population of peptides is identified by screening a library of peptides of about 8 to about 180 amino acids encoded by nucleic acid fragments obtainable from genome(s) of a microorganisms and/or a eukaryotic species having a compact genome, or of nucleic acids that encode peptides of about 8 to about 180 amino acids encoded by nucleic acid fragments obtainable from genome(s) of a microorganisms and/or a eukaryotic species having a compact genome, for binding of said encoded peptides to said target protein;
empirically determining the binding configuration of a plurality of binding peptides, within said population that has different sequences, to said target protein, wherein at least one of said binding peptides modulates the phenotype in a mammalian cell capable of displaying the phenotype; and
identifying: (i) locations of binding energy; and/or (ii) the orientation and identity of at least one side chain of said plurality of binding peptides that interacts with said protein target, in either case characterising the three dimensional structure of said interaction site by analysis of said binding configuration, wherein said three dimensional structure is characterised using *in silico* methods, and
(x) docking the structure of a small molecule to the characterised interaction site *in silico*; or
(y) employing at least one of said binding peptides and the target protein in an assay to identify a small molecule that modulates the binding of the peptide to the target protein at the interaction site,
thereby characterising the interaction site on said target protein as dockable by a small molecule ligand.

2. The method of claim 1 comprising step (x) of docking the structure of a small molecule to the characterised interaction site, and further comprising the step of:
(z) providing one or more of said binding peptides and determining the ability of said small molecule to compete with one or more of said peptides for binding to said target protein.

3. The method according to claim 1 or 2, wherein the phenotype of a mammalian cell is:
one associated with a cell signalling pathway, preferably an activated cell signalling pathway; and/or one selected from the list consisting of: differentiation, migration, invasion, chemotaxis, immunological anergy, surface marker expression, transcriptional activity, protein expression, glycosylation, resistance to infection, permeability and reporter-gene activity.

4. The method according to any one of claims 1 to 3, wherein: (i) said library of peptides comprises a plurality of separate and addressable peptides; or (ii) said library of peptides is expressed from a plurality of separate and addressable nucleic acids that encode peptides in said library.

5. The method according to any one of claims 1 to 3, wherein: (i) said library of peptides comprises a pooled plurality of peptides; or (ii) said library of peptides is expressed from a pooled plurality of nucleic acids that encode peptides in said library.

6. The method according to any one of claims 1 to 5, wherein:
(i) said library of peptides comprises 3 x 10⁴ or more, such as 1 x 10⁶ or more, different amino acid sequences; or
(ii) said library of peptides is expressed from a plurality of nucleic acids comprising 3 x 10⁴ or more, such as 1 x 10⁶ or more, different nucleic acid sequences that encode peptides in said library; and/or
(a) said library of peptides comprises 3 x 10⁴ or more, such as 1 x 10⁶ or more, different peptides; or
(b) said library of peptides is expressed from a plurality of nucleic acids that encode 3 x 10⁴ or more, such as 1 x 10⁶ or more, different peptides in said library.

7. The method according to any one of claims 1 to 6 comprising isolating, from said population of mammalian cells, said cell which displays said alteration in phenotype following exposure to said library of peptides.

8. The method according to any one of claims 1 to 7, wherein said provided identified peptide is provided as part of a fusion protein that comprises the identified peptide and an affinity tag; and/or wherein said cellular protein is identified by mass spectrometry or by protein-microarray analysis with said provided identified peptide.

9. The method according to any one of claims 1 to 8, wherein:
(a) said cellular protein is identified using standard screens for cellular binding; optionally wherein: (i) in said screen, said provided identified peptide is used as a bait molecule to identify said cellular protein in a mammalian cell or cell extract; and/or; (ii) said screen is selected from the list consisting of: phage or ribosome display and two-hybrid screening; preferably, yeast or mammalian cell-based or in-vitro two-hybrid screening; and/or
(b) said method comprises, prior to identification of said cellular protein, isolating a cellular protein which binds to said provided identified peptide; *optionally* using a technique selected from the list consisting of: radioimmunoassay, co-immunoprecipitation, two-hybrid techniques, the probing of arrays of candidate proteins using labelled peptides from said library, scintillation proximity assays and ELISA methods; *preferably* wherein, the provided identified peptide is over-expressed in mammalian cells and immunoprecipitated with antibodies binding to an epitope tag.

10. The method according to any one of claims 1 to 9 comprising testing at least one of the peptides from said provided population of peptides for its ability to modulate said phenotype of a mammalian cell capable of displaying said phenotype;
*preferably* wherein said testing comprises exposing a mammalian cell capable of displaying said phenotype to said at least one peptide and determining if said phenotype of said mammalian cell is modulated.

11. The method according to any one of claims 1 to 10, wherein said provided target protein is provided in isolated form, optionally wherein said isolated target protein is immobilised, or said provided target protein is provided from expression of a nucleotide sequence encoding said target protein in a host cell.

12. The method according to any one of claims 1 to 11, wherein said provided population of peptides which bind to the target protein comprises at least 5 peptides.

13. The method according to any one of claims 1 to 12 comprising identifying a sub-population of peptides within said provided population of peptides, where said sub-population comprises peptides of different sequences, and/or
measuring the binding affinity to said target protein of at least one of the peptides in said provided population of peptides.

## Patentansprüche

1. Verfahren zur Charakterisierung einer Interaktionsstelle an einem Zielprotein als andockbar durch einen niedermolekularen Liganden, wobei das Zielprotein einen Phänotyp einer Säugetierzelle moduliert, wobei das Verfahren die folgenden Schritte umfasst:
Aussetzen einer Population von *in vitro* kultivierten Säugetierzellen, die den Phänotyp anzeigen können, einer Peptidbibliothek von etwa 8 bis etwa 180 Aminosäuren, kodiert durch Nukleinsäurefragmente, die von Genom(en) eines Mikroorganismus und/oder einer eukaryotischen Spezies mit einem kompakten Genom erhalten werden können;
Identifizieren einer Zelle in der Population, die eine nach der Exposition eine Veränderung in dem Phänotyp anzeigt;
Identifizieren eines Peptids aus der Peptidbibliothek, das den Phänotyp der Zelle verändert;
Bereitstellen des identifizierten Peptids;
Identifizieren eines zellulären Proteins, das an das bereitgestellte identifizierte Peptid bindet, wobei das zelluläre Protein ein Zielprotein ist, das den Phänotyp der Säugetierzelle moduliert;
Bereitstellen des Zielproteins;
Bereitstellen einer Population von Peptiden von etwa 8 bis etwa 180 Aminosäuren, kodiert durch Nukleinsäurefragmente, die von Genom(en) eines Mikroorganismus und/oder einer eukaryotischen Spezies mit einem kompakten Genom erhalten werden können, wobei die Peptide in der Population an der Interaktionsstelle an das Zielprotein binden, wobei die Population von Peptiden vor deren Bereitstellung identifiziert wird durch Screening einer Peptidbibliothek von etwa 8 bis etwa 180 Aminosäuren, kodiert durch Nukleinsäurefragmente, die von Genom(en) eines Mikroorganismus und/oder einer eukaryotischen Spezies mit einem kompakten Genom erhalten werden können, oder von Nukleinsäuren, die für Peptide von etwa 8 bis 180 Aminosäuren kodieren, kodiert durch Nukleinsäurefragmente, die von Genom(en) eines Mikroorganismus und/oder einer eukaryotischen Spezies mit einem kompakten Genom erhalten werden können, zur Bindung der kodierten Peptide an das Zielprotein;
empirisches Bestimmen der Bindungsgestalt einer Mehrzahl von Bindungspeptiden in der Population, die unterschiedliche Sequenzen aufweist, an das Zielprotein, wobei mindestens eines der Bindungspeptide den Phänotyp in einer Säugetierzelle moduliert, die den Phänotyp anzeigen kann; und
Identifizieren: (i) von Stellen mit Bindungsenergie; und/oder (ii) der Ausrichtung und Identität mindestens einer Seitenkette der Mehrzahl von Bindungspeptiden, die mit dem Proteinziel interagiert, wobei in jedem Fall die dreidimensionale Struktur der Interaktionsstelle durch Analyse der Bindungsgestalt charakterisiert wird, wobei die dreidimensionale Struktur unter Verwendung von *in silico*-Verfahren charakterisiert wird, und
(x) Docking der Struktur einer niedermolekularen Substanz an die charakterisierte Interaktionsstelle *in silico*; oder
(y) Einsetzen mindestens eines der Bindungspeptide und des Zielproteins in einem Assay, um eine niedermolekulare Substanz zu identifizieren, welche die Bindung des Peptids an das Zielprotein an der Interaktionsstelle moduliert,
wodurch die Interaktionsstelle an dem Zielprotein als andockbar durch einen niedermolekularen Liganden charakterisiert wird.

2. Verfahren nach Anspruch 1, umfassend den Schritt (x) des Dockings der Struktur einer niedermolekularen Substanz an die charakterisierte Interaktionsstelle, und wobei das Verfahren ferner den folgenden Schritt umfasst:
(z) Bereitstellen eines oder mehrere der Bindungspeptide und Bestimmen der Fähigkeit der niedermolekularen Substanz, mit einem oder mehreren der Peptide um die Bindung an das Zielprotein zu konkurrieren.

3. Verfahren nach Anspruch 1 oder 2, wobei der Phänotyp einer Säugetierzelle folgendes ist:
ein Phänotyp assoziiert mit einem Zellsignalisierungsweg, vorzugsweise einem Signalisierungsweg einer aktivierten Zelle; und/oder ein Phänotyp, der ausgewählt ist aus der Liste bestehend aus: Differenzierung, Migration, Invasion, Chemotaxis, immunologischer Anergie, Oberflächenmarker-Expression, Transkriptionsaktivität, Proteinexpression, Glykosylierung, Infektionsresistenz, Permeabilität und Reportergenaktivität.

4. Verfahren nach einem der Ansprüche 1 bis 3, wobei: (i) die Peptidbibliothek eine Mehrzahl separater und adressierbarer Peptide umfasst; oder (ii) die Peptidbibliothek aus einer Mehrzahl separater und adressierbare Nukleinsäuren, die für Peptide in der Bibliothek kodieren, exprimiert ist.

5. Verfahren nach einem der Ansprüche 1 bis 3, wobei: (i) die Peptidbibliothek eine gepoolte Mehrzahl von Peptiden umfasst; oder (ii) die Peptidbibliothek aus einer gepoolten Mehrzahl von Nukleinsäuren, die für Peptide in der Bibliothek kodieren, exprimiert ist.

6. Verfahren nach einem der Ansprüche 1 bis 5, wobei:
(i) die Peptidbibliothek 3 x 10⁴ oder mehr, wie etwa 1 x 10⁶ oder mehr, verschiedene Aminosäuresequenzen umfasst; oder
(ii) die Peptidbibliothek exprimiert ist aus einer Mehrzahl von Nukleinsäuren, die 3 x 10⁴ oder mehr, wie etwa 1 x 10⁶ oder mehr, verschiedene Aminosäuresequenzen umfassen, welche für die Peptide in der Bibliothek kodieren; und/oder
(a) die Peptidbibliothek umfasst 3 x 10⁴ oder mehr, wie etwa 1 x 10⁶ oder mehr, verschiedene Peptide; oder
(b) die Peptidbibliothek ist exprimiert aus einer Mehrzahl von Nukleinsäuren, die für 3 x 10⁴ oder mehr, wie etwa 1 x 10⁶ oder mehr, verschiedene in der Bibliothek kodieren.

7. Verfahren nach einem der Ansprüche 1 bis 6, umfassend das Isolieren der Population von Säugetierzellen, wobei die Zelle die Veränderung des Phänotyps anzeigt, nachdem sie der Peptidbibliothek ausgesetzt worden ist.

8. Verfahren nach einem der Ansprüche 1 bis 7, wobei das bereitgestellte identifizierte Peptid bereitgestellt ist als Teil eines Fusionsproteins, welches das identifizierte Peptid und ein Affinitäts-Tag umfasst; und/oder wobei das zelluläre Protein identifiziert wird durch Massenspektrometrie oder durch Protein-Mikroarray-Analyse mit dem bereitgestellten identifizierten Peptid.

9. Verfahren nach einem der Ansprüche 1 bis 8, wobei:
(a) das zelluläre Protein identifiziert wird durch Standard-Screenings für zelluläre Bindung; wobei optional: (i) bei dem Screening das bereitgestellte identifizierte Peptid als ein Ködermolekül verwendet wird, um das zelluläre Protein in einer Säugetierzelle oder einem Zellextrakt zu identifizieren; und/oder; (ii) das Screening ausgewählt ist aus der Liste bestehend aus: Phagen- oder Ribosomen-Display und Zwei-Hybrid-Screening; vorzugsweise Hefe- oder Säugetierzellen-basiertem oder in vitro Zwei-Hybrid-Screening; und/oder
(b) das Verfahren vor der Identifizierung des zellulären Proteins das Isolieren eines zellulären Proteins umfasst, das an das bereitgestellte identifizierte Peptid bindet; *optional* unter Verwendung einer Technik, die ausgewählt ist aus der Liste bestehend aus: Radioimmunassay, Co-Immunpräzipitation, Zwei-Hybrid-Techniken, der Prüfung von Arrays von Kandidatenproteinen unter Verwendung markierter Peptide aus der Bibliothek, Scintillation Proximity Assays und ELISA-Methoden; wobei das bereitgestellte identifizierte Peptid *vorzugsweise* in Säugetierzellen überexprimiert ist und immunpräzipitiert mit an ein Epitop-Tag bindenden Antikörpern.

10. Verfahren nach einem der Ansprüche 1 bis 9, umfassend das Testen mindestens eines der Peptide aus der bereitgestellten Population von Peptiden auf dessen Fähigkeit, den Phänotyp einer Säugetierzelle zu modulieren, die den Phänotyp anzeigen kann;
wobei es das Testen *vorzugsweise* umfasst, dass eine Säugetierzelle, die den Phänotyp anzeigen kann, dem mindestens einen Peptid ausgesetzt wird, und das Bestimmen, ob der Phänotyp der Säugetierzelle moduliert ist.

11. Verfahren nach einem der Ansprüche 1 bis 10, wobei das bereitgestellte Zielprotein in isolierter Form bereitgestellt wird, wobei das isolierte Zielprotein optional immobilisiert wird, oder wobei das bereitgestellte Zielprotein bereitgestellt wird aus der Expression einer für das Zielprotein in einer Wirtszelle kodierenden Nukleotidsequenz.

12. Verfahren nach einem der Ansprüche 1 bis 11, wobei die bereitgestellte Population von Peptiden, die an das Zielprotein binden, mindestens 5 Peptide umfasst.

13. Verfahren nach einem der Ansprüche 1 bis 12, umfassend das Identifizieren einer Unterpopulation von Peptiden in der bereitgestellten Population von Peptiden, wobei die Unterpopulation Peptide verschiedener Sequenzen umfasst, und/oder
Messen der Bindungsaffinität mindestens eines der Peptide in der bereitgestellten Population von Peptiden an das Zielprotein.

## Revendications

1. Procédé de caractérisation d'un site d'interaction sur une protéine cible comme ancrable par un ligand à petite molécule, la protéine cible modulant un phénotype d'une cellule mammalienne, ledit procédé comprenant les étapes consistant à :
exposer une population de cellules mammaliennes cultivées *in-vitro* capables d'afficher ledit phénotype à une banque de peptides d'environ 8 à environ 180 acides aminés codés par des fragments d'acides nucléiques pouvant être obtenus à partir d'un ou de plusieurs génomes d'un microorganisme et/ou d'une espèce eucaryote ayant un génome compact ;
identifier une cellule dans la population qui affiche une modification dans ledit phénotype à la suite de ladite exposition ;
identifier un peptide à partir de ladite banque de peptides qui modifie ledit phénotype de la cellule ;
fournir le peptide identifié ;
identifier une protéine cellulaire qui se lie audit peptide identifié fourni, ladite protéine cellulaire étant une protéine cible qui module ledit phénotype de la cellule mammalienne ;
fournir ladite protéine cible ;
fournir une population de peptides d'environ 8 à environ 180 acides aminés codés par des fragments d'acides nucléiques pouvant être obtenus à partir d'un ou de plusieurs génomes d'un microorganisme et/ou d'une espèce eucaryote ayant un génome compact, les peptides dans ladite population se liant à ladite protéine cible au niveau du site d'interaction, avant sa fourniture, ladite population de peptides étant identifiée par criblage d'une banque de peptides d'environ 8 à environ 180 acides aminés codés par des fragments d'acides nucléiques pouvant être obtenus à partir d'un ou de plusieurs génomes d'un microorganisme et/ou d'une espèce eucaryote ayant un génome compact, ou d'acides nucléiques qui codent des peptides d'environ 8 à environ 180 acides aminés codés par des fragments d'acides nucléiques pouvant être obtenus à partir d'un ou de plusieurs génomes d'un microorganismes et/ou d'une espèce eucaryote ayant un génome compact, pour une liaison desdits peptides codés à ladite protéine cible ;
déterminer empiriquement la configuration de liaison d'une pluralité de peptides de liaison, au sein de ladite population qui a différentes séquences, à ladite protéine cible, au moins un desdits peptides de liaison modulant le phénotype dans une cellule mammalienne capable d'afficher le phénotype ; et
identifier : (i) des emplacements d'énergie de liaison ; et/ou (ii) l'orientation et l'identité d'au moins une chaîne latérale de ladite pluralité de peptides de liaison qui interagit avec ladite cible protéine, dans chaque cas caractérisant la structure tridimensionnelle dudit site d'interaction par analyse de ladite configuration de liaison, ladite structure tridimensionnelle étant **caractérisée** en utilisant des méthodes *in silico,* et
(x) ancrer la structure d'une petite molécule au site d'interaction caractérisé *in silico* ; ou
(y) employer au moins un desdits peptides de liaison et la protéine cible dans un dosage afin d'identifier une petite molécule qui module la liaison du peptide à la protéine cible au niveau du site d'interaction,
ce qui permet de caractériser le site d'interaction sur ladite protéine cible comme ancrable par un ligand à petite molécule.

2. Procédé selon la revendication 1, comprenant l'étape (x) consistant à ancrer la structure d'une petite molécule au site d'interaction caractérisé, et comprenant en outre l'étape consistant à :
(z) fournir un ou plusieurs desdits peptides de liaison et déterminer la capacité de ladite petite molécule à concurrencer un ou plusieurs desdits peptides pour une liaison à ladite protéine cible.

3. Procédé selon la revendication 1 ou 2, le phénotype d'une cellule mammalienne étant :
un associé à une voie de signalisation cellulaire, de préférence une voie de signalisation cellulaire activée ; et/ou un sélectionné dans la liste constituée par : différenciation, migration, invasion, chimiotaxie, anergie immunologique, expression de marqueur de surface, activité transcriptionnelle, expression de protéine, glycosylation, résistance à l'infection, perméabilité et activité de gène rapporteur.

4. Procédé selon l'une quelconque des revendications 1 à 3 : (i) ladite banque de peptides comprenant une pluralité de peptides séparés et adressables ; ou (ii) ladite banque de peptides étant exprimée à partir d'une pluralité d'acides nucléiques séparés et adressables qui codent des peptides dans ladite banque.

5. Procédé selon l'une quelconque des revendications 1 à 3 : (i) ladite banque de peptides comprenant une pluralité groupée de peptides ; ou (ii) ladite banque de peptides étant exprimée à partir d'une pluralité groupée d'acides nucléiques qui codent des peptides dans ladite banque.

6. Procédé selon l'une quelconque des revendications 1 à 5 :
(i) ladite banque de peptides comprenant 3 x 10⁴ ou plus, tel que 1 x 10⁶ ou plus, séquences d'acides aminés différentes ; ou
(ii) ladite banque de peptides étant exprimée à partir d'une pluralité d'acides nucléiques comprenant 3 x 10⁴ ou plus, tel que 1 x 10⁶ ou plus, séquences d'acides nucléiques différentes qui codent des peptides dans ladite banque ; et/ou
(a) ladite banque de peptides comprenant 3 x 10⁴ ou plus, tel que 1 x 10⁶ ou plus, peptides différents ; ou
(b) ladite banque de peptide étant exprimée à partir d'une pluralité d'acides nucléiques qui codent 3 x 10⁴ ou plus, tel que 1 x 10⁶ ou plus, peptides différents dans ladite banque.

7. Procédé selon l'une quelconque des revendications 1 à 6, comprenant l'étape consistant à isoler, à partir de ladite population de cellules mammaliennes, ladite cellule qui affiche ladite modification dans le phénotype à la suite d'une exposition à ladite banque de peptides.

8. Procédé selon l'une quelconque des revendications 1 à 7, ledit peptide identifié fourni étant fourni en tant que partie d'une protéine de fusion qui comprend le peptide identifié et une étiquette d'affinité ; et/ou ladite protéine cellulaire étant identifiée par spectrométrie de masse ou par analyse par microréseaux de protéines avec ledit peptide identifié fourni.

9. Procédé selon l'une quelconque des revendications 1 à 8 :
(a) ladite protéine cellulaire étant identifiée en utilisant des cribles standards pour la liaison cellulaire ; éventuellement : (i) dans ledit crible, ledit peptide identifié fourni étant utilisé en tant que molécule leurre afin d'identifier ladite protéine cellulaire dans une cellule ou un extrait de cellule mammalienne ; et/ou (ii) ledit crible étant sélectionné dans la liste constituée par : affichage sur phage ou ribosome et criblage par double hybride ; de préférence criblage par double hybride in-vitro ou à base de cellule mammalienne ou de levure ; et/ou
(b) ledit procédé comprenant, avant l'identification de ladite protéine cellulaire, l'étape consistant à isoler une protéine cellulaire qui se lie audit peptide identifié fourni ; *éventuellement* en utilisant une technique sélectionnée dans la liste constituée par : un dosage radio-immunologique, une co-immunoprécipitation, des techniques par double hybride, le sondage de réseaux de protéines candidates en utilisant des peptides marqués issus de ladite banque, des dosages de la scintillation par proximité et des procédés ELISA ; *de préférence,* le peptide identifié fourni étant sur-exprimé dans des cellules mammaliennes et immunoprécipité avec des anticorps se liant à une étiquette d'épitope.

10. Procédé selon l'une quelconque des revendications 1 à 9, comprenant l'étape consistant à tester au moins un des peptides à partir de ladite population fournie de peptides pour sa capacité à moduler ledit phénotype d'une cellule mammalienne capable d'afficher ledit phénotype ;
*de préférence* ladite étape consistant à tester comprenant les étapes consistant à exposer une cellule mammalienne capable d'afficher ledit phénotype audit au moins un peptide et déterminer si ledit phénotype de ladite cellule mammalienne est modulé.

11. Procédé selon l'une quelconque des revendications 1 à 10, ladite protéine cible fournie étant fournie sous forme isolée, éventuellement ladite protéine cible isolée étant immobilisée, ou ladite protéine cible fournie étant fournie à partir de l'expression d'une séquence nucléotidique codant ladite protéine cible dans une cellule hôte.

12. Procédé selon l'une quelconque des revendications 1 à 11, ladite population fournie de peptides qui se lient à la protéine cible comprenant au moins 5 peptides.

13. Procédé selon l'une quelconque des revendications 1 à 12, comprenant les étapes consistant à identifier une sous-population de peptides au sein de ladite population fournie de peptides, ladite sous-population comprenant des peptides de différentes séquences, et/ou
mesurer l'affinité de liaison à ladite protéine cible d'au moins un des peptides dans ladite population fournie de peptides.


### SEQUENCE LISTING

<110> Phylogica Limited Cambridge Enterprise Limited
<120> Methods for the characterisation of interaction sites in target proteins
<130> 158397
<150> EP12154872.1
   <151> 10-02-2012
<160> 20
<170> BISSAP 1.0
<210> 1
   <211> 40
   <212> DNA
   <213> null
<220>
   <221> source
   <222> 1..40
   <223> /mol_type="DNA" /organism=null
<400> 1
   gactacaagg acgacgacga caaggcttat caatcaatca 40
<210> 2
   <211> 40
   <212> DNA
   <213> null
<220>
   <221> source
   <222> 1..40
   <223> /mol_type="DNA" /organism=null
<400> 2
   gactacaagg acgacgacga caaggcttat caatcaatca 40
<210> 3
   <211> 41
   <212> DNA
   <213> null
<220>
   <221> source
   <222> 1..41
   <223> /mol_type="DNA" /organism=null
<400> 3
   gagaggaatt caggtcagac tacaaggacg acgacgacaa g 41
<210> 4
   <211> 41
   <212> DNA
   <213> null
<220>
   <221> source
   <222> 1..41
   <223> /mol_type="DNA" /organism=null
<400> 4
   gagagggtac caggtcagac tacaaggacg acgacgacaa g 41
<210> 5
   <211> 15
   <212> PRT
   <213> null
<220>
   <221> SOURCE
   <222> 1..15
   <223> /mol_type="protein" /organism=null
<400> 5
   Gly Leu Gln Gly Arg Arg Arg Gln Gly Tyr Gln Ser Ile Lys Pro
   1               5                   10                  15
<210> 6
   <211> 166
   <212> PRT
   <213> null
<220>
   <221> SOURCE
   <222> 1..166
   <223> /mol_type="protein" /organism=null
<400> 6
   Leu Lys His Arg Asp Tyr Trp Val Pro Leu Leu Tyr Phe Phe Phe Val
   1               5                   10                  15
   Leu Ile Thr Met Phe Trp Asp Leu Tyr Cys His Ser Gly Gln Tyr Leu
               20                  25                  30
   Tyr Glu Ala Arg Ser Leu Leu Pro Phe Tyr Leu Phe Pro Phe Leu Ile
           35                  40                  45
   Ala Ala Leu Arg Ile Gly Asp Ile Val Val Asp Thr Arg Cys Arg Asn
       50                  55                  60
   Val Glu Asn Leu Gly Gly Phe Glu Arg Ser Ala Glu Ser Ser Arg Tyr
   65                  70                  75                  80
   Val Gln Leu Ser Leu Tyr Glu Arg Gln Trp Phe Arg Leu Arg Tyr Arg
                   85                  90                  95
   Ala Tyr Gly Gly Asn Leu Arg Cys Leu Ala Gly Asp Leu Val Glu Asn
               100                 105                 110
   Trp Arg Leu Trp Lys Glu Val Asn Ser Lys Ala Ala Lys Asp Ile Asp
           115                 120                 125
   Gly Ala Val Met Leu Arg Phe Phe Leu Pro Pro Arg Asp Leu Ala Arg
       130                 135                 140
   Leu Val Thr Leu Leu Ala Ala Leu Ile Ala Val Ile Ala Thr Leu Ile
   145                 150                 155                 160
   Ile Thr Leu Ala Ile Val
                   165
<210> 7
   <211> 501
   <212> DNA
   <213> null
<220>
   <221> source
   <222> 1..501
   <223> /mol_type="DNA" /organism=null
<400> 7
   ctgaaacaca gggactactg ggttcccctg ctgtatttct tcttcgtgtt gatcacgatg      60
   ttctgggact tgtattgcca ctccggacag tacctctacg aagccaggag tctgctccct     120
   ttctacctgt tcccgtttct gatcgctgcg ctacggatag gcgatatcgt cgtcgatacg     180
   cgctgtcgca atgtggagaa cctgggaggc ttcgagcgct ccgccgagag cagtcgctac     240
   gtccagcttt ccctctacga acggcaatgg ttccggctgc ggtaccgggc ctatggcggt     300
   aacctccgct gcctggcggg cgatctggtg gagaactggc gcctctggaa ggaggtgaat     360
   tcgaaggccg cgaaggatat cgacggcgca gtgatgctgc gattctttct gcctccccgg     420
   gaccttgctc gtctggtcac gctgttggct gcgctcattg cggtcatcgc cactctcatc     480
   attaccctgg caattgtatg a                                               501
<210> 8
   <211> 20
   <212> PRT
   <213> null
<220>
   <221> SOURCE
   <222> 1..20
   <223> /mol_type="protein" /organism=null
<400> 8
   Pro His Arg His Asn Arg Leu Ala Leu Arg Cys Tyr Leu Thr His Arg
   1               5                   10                  15
   Leu Thr Arg Leu
               20
<210> 9
   <211> 63
   <212> DNA
   <213> null
<220>
   <221> source
   <222> 1..63
   <223> /mol_type="DNA" /organism=null
<400> 9
   ccacaccgtc ataatcgcct tgctcttcgg tgttatctga cccatcgtct gacacgtttg 60
   taa 63
<210> 10
   <211> 9
   <212> PRT
   <213> null
<220>
   <221> SOURCE
   <222> 1..9
   <223> /mol_type="protein" /organism=null
<400> 10
   Arg Ala Glu Lys Cys Gly Lys Leu Phe
   1 5
<210> 11
   <211> 30
   <212> DNA
   <213> null
<220>
   <221> source
   <222> 1..30
   <223> /mol_type="DNA" /organism=null
<400> 11
   cgggcagaaa aatgcgggaa gctgttctga 30
<210> 12
   <211> 15
   <212> PRT
   <213> null
<220>
   <221> SOURCE
   <222> 1..15
   <223> /mol_type="protein" /organism=null
<400> 12
   Pro Arg Arg His Gly Asn Gly Ser Pro Ser Leu Phe His Gly Arg
   1 5 10 15
<210> 13
   <211> 48
   <212> DNA
   <213> null
<220>
   <221> source
   <222> 1..48
   <223> /mol_type="DNA" /organism=null
<400> 13
   ccccgaagac atggtaacgg atcgccctct ctctttcatg gccgctga 48
<210> 14
   <211> 16
   <212> PRT
   <213> null
<220>
   <221> SOURCE
   <222> 1..16
   <223> /mol_type="protein" /organism=null
<400> 14
   Leu Gly Ala Ala Gly Pro Thr His Tyr Asp His Leu Asp Cys Ala Arg
   1 5 10 15
<210> 15
   <211> 51
   <212> DNA
   <213> null
<220>
   <221> source
   <222> 1..51
   <223> /mol_type="DNA" /organism=null
<400> 15
   ctcggagcgg cggggccaac gcattatgat cacctcgact gcgcccggtg a 51
<210> 16
   <211> 9
   <212> PRT
   <213> null
<220>
   <221> SOURCE
   <222> 1..9
   <223> /mol_type="protein" /organism=null
<400> 16
   Pro Leu Pro Phe Pro Ser Pro Val Pro
   1 5
<210> 17
   <211> 30
   <212> DNA
   <213> null
<220>
   <221> source
   <222> 1..30
   <223> /mol_type="DNA" /organism=null
<400> 17.
   ccgttgccgt tcccaagtcc ggtgccgtga 30
<210> 18
   <211> 8
   <212> PRT
   <213> null
<220>
   <221> SOURCE
   <222> 1..8
   <223> /mol_type="protein" /organism=null
<400> 18
   His Ser Asp Arg Thr Ala Asp Ile
   1               5
<210> 19
   <211> 27
   <212> DNA
   <213> null
<220>
   <221> source
   <222> 1..27
   <223> /mol_type="DNA" /organism=null
<400> 19
   cactcagata gaacagcaga tatttga 27
<210> 20
   <211> 18
   <212> PRT
   <213> null
<220>
   <221> SOURCE
   <222> 1..18
   <223> /mol_type="protein" /organism=null
<400> 20
   Ala Tyr Gln Ser Ile Arg Ser Gly Gly Ile Glu Ser Ser Ser Lys Arg
   1               5                   10                  15
   Glu Arg